# FASCICULE DE BREVET EUROPEEN

(11) **EP 2 788 375 B1**
(45) Date de publication et mention de la délivrance du brevet: **12.07.2017**
(21) Numéro de dépôt: 12810334.8
(22) Date de dépôt: 04.12.2012
(51) Int. Cl.: C07K 16/08, C07K 14/005, C12N 7/00, C12Q 1/70, G01N 33/569

(54) **UTILISATION D'AU MOINS UN BIOMARQUEUR POUR LE PRONOSTIC OU LE DIAGNOSTIC IN VITRO D'EPISODES LYMPHOPROLIFERATIFS ASSOCIES AU VIRUS D'EPSTEIN-BARR (EBV)**
VERWENDUNG MINDESTENS EINES BIOMARKERS ZUR IN-VITRO-PROGNOSE LYMPHOPROLIFERATIVER EPISODEN MIT DEM EPSTEIN-BARR-VIRUS (EBV)
USE OF AT LEAST ONE BIOMARKER FOR THE IN VITRO PROGNOSIS OR DIAGNOSIS OF LYMPHOPROLIFERATIVE EPISODES ASSOCIATED WITH THE EPSTEIN-BARR VIRUS (EBV)

(30) Priorité: 05.12.2011 FR 1161167
(43) Date de publication de la demande: 15.10.2014
(73) Titulaire: Université Grenoble Alpes, 38401 Saint-Martin-d'Hères (FR)
(72) Inventeur: DROUET, Emmanuel, 38700 Corenc (FR); HABIB, Mohammed, 42000 Saint Etienne (FR); AGBALIKA, Félix, 75020 Paris (FR)
(74) Mandataire: Grosset-Fournier, Chantal Catherine
(86) Numéro de dépôt international: PCT/FR2012/052790
(87) Numéro de publication internationale: WO 2013/083906

(56) Documents cités:
- WO-A1-96/21155
- WO-A1-2009/112497
- US-A1- 2004 214 161
- YOUNG ET AL: "Differentiation-associated expression of the Epstein-Barr virus BZLF1 transactivator protein in oral hairy leukoplakia.", JOURNAL OF VIROLOGY, vol. 65, no. 6, 1 juin 1991 (1991-06-01), pages 2868-2874, XP055054885, ISSN: 0022-538X
- D. A. KATZ: "Viral Proteins Associated with the Epstein-Barr Virus Transactivator, ZEBRA", PROCEEDINGS OF THE NATIONAL ACADEMY OF SCIENCES, vol. 89, no. 1, 1 janvier 1992 (1992-01-01), pages 378-382, XP055037357, ISSN: 0027-8424, DOI: 10.1073/pnas.89.1.378 cité dans la demande
- M. TARDIF: "Impaired Protein Kinase C Activation/Translocation in Epstein-Barr Virus-infected Monocytes", JOURNAL OF BIOLOGICAL CHEMISTRY, vol. 277, no. 27, 28 juin 2002 (2002-06-28), pages 24148-24154, XP055037364, ISSN: 0021-9258, DOI: 10.1074/jbc.M109036200 cité dans la demande
- Y. AL TABAA ET AL: "Functional Epstein-Barr virus reservoir in plasma cells derived from infected peripheral blood memory B cells", BLOOD, vol. 113, no. 3, 15 janvier 2009 (2009-01-15), pages 604-611, XP055037274, ISSN: 0006-4971, DOI: 10.1182/blood-2008-02-136903
- TAN ENG-LAI ET AL: "Quantification of Epstein-Barr virus DNA load, interleukin-6, interleukin-10, transforming growth factor-[beta]1 and stem cell factor in plasma of patients with nasopharyngeal carcinoma", BMC CANCER, BIOMED CENTRAL, LONDON, GB, vol. 6, no. 1, 24 septembre 2006 (2006-09-24), page 227, XP021016225, ISSN: 1471-2407, DOI: 10.1186/1471-2407-6-227
- DARDARI ET AL: "Analyses of the prognostic significance of the Epstein-Barr virus transactivator ZEBRA protein and diagnostic value of its two synthetic peptides in nasopharyngeal carcinoma", JOURNAL OF CLINICAL VIROLOGY, ELSEVIER, AMSTERDAM, NL, vol. 41, no. 2, 19 novembre 2007 (2007-11-19), pages 96-103, XP022503936, ISSN: 1386-6532, DOI: 10.1016/J.JCV.2007.09.010
- C. HILSCHER: "Faster quantitative real-time PCR protocols may lose sensitivity and show increased variability", NUCLEIC ACIDS RESEARCH, vol. 33, no. 21, 27 novembre 2005 (2005-11-27), pages e182-e182, XP055037375, ISSN: 0305-1048, DOI: 10.1093/nar/gni181
- R. M. WADOWSKY ET AL: "Measurement of Epstein-Barr Virus DNA Loads in Whole Blood and Plasma by TaqMan PCR and in Peripheral Blood Lymphocytes by Competitive PCR", JOURNAL OF CLINICAL MICROBIOLOGY, vol. 41, no. 11, 1 novembre 2003 (2003-11-01), pages 5245-5249, XP055037377, ISSN: 0095-1137, DOI: 10.1128/JCM.41.11.5245-5249.2003

### SEQUENCE LISTING

<110> Université Joseph Fourier
   Assistance Publique-Hopitaux de Paris
   Drouet, Emmanuel
   Habib, Mohammed
   Agbalika, Félix
<120> UTILISATION D'AU MOINS UN BIOMARQUEUR POUR LE PRONOSTIC OU LE DIAGNOSTIC IN VITRO D'EPISODES LYMPHOPROLIFERATIFS ASSOCIES AU VIRUS D'EPSTEIN-BARR (EBV)
<130> IFB 11 CI UJF EBV+
<160> 8
<170> PatentIn version 3.5
<210> 1
   <211> 245
   <212> PRT
   <213> Epstein-Barr virus
<400> 1
   Met Met Asp Pro Asn Ser Thr Ser Glu Asp Val Lys Phe Thr Pro Asp
   1               5                   10                  15
   Pro Tyr Gln Val Pro Phe Val Gln Ala Phe Asp Gln Ala Thr Arg Val
               20                  25                  30
   Tyr Gln Asp Leu Gly Gly Pro Ser Gln Ala Pro Leu Pro Cys Val Leu
           35                  40                  45
   Trp Pro Val Leu Pro Glu Pro Leu Pro Gln Gly Gln Leu Thr Ala Tyr
       50                  55                  60
   His Val Ser Thr Ala Pro Thr Gly Ser Trp Phe Ser Ala Pro Gln Pro
   65                  70                  75                  80
   Ala Pro Glu Asn Ala Tyr Gln Ala Tyr Ala Ala Pro Gln Leu Phe Pro
                   85                  90                  95
   Val Ser Asp Ile Thr Gln Asn Gln Gln Thr Asn Gln Ala Gly Gly Glu
               100                 105                 110
   Ala Pro Gln Pro Gly Asp Asn Ser Thr Val Gln Thr Ala Ala Ala Val
           115                 120                 125
   Val Phe Ala Cys Pro Gly Ala Asn Gln Gly Gln Gln Leu Ala Asp Ile
       130                 135                 140
   Gly Val Pro Gln Pro Ala Pro Val Ala Ala Pro Ala Arg Arg Thr Arg
   145                 150                 155                 160
   Lys Pro Gln Gln Pro Glu Ser Leu Glu Glu Cys Asp Ser Glu Leu Glu
                   165                 170                 175
   Ile Lys Arg Tyr Lys Asn Arg Val Ala Ser Arg Lys Cys Arg Ala Lys
               180                 185                 190
   Phe Lys Gln Leu Leu Gln His Tyr Arg Glu Val Ala Ala Ala Lys Ser
           195                 200                 205
   Ser Glu Asn Asp Arg Leu Arg Leu Leu Leu Lys Gln Met Cys Pro Ser
       210                 215                 220
   Leu Asp Val Asp Ser Ile Ile Arg Arg Thr Pro Asp Val Leu His Glu
   225                 230                 235                 240
   Asp Leu Leu Asn Phe
                   245
<210> 2
   <211> 19
   <212> DNA
   <213> Artificial Sequence
<220>
   <223> BALF-5_F
<400> 2
   cggaagccct ctggacttc 19
<210> 3
   <211> 21
   <212> DNA
   <213> Artificial Sequence
<220>
   <223> BALF-5_R
<400> 3
   ccctgtttat ccgatggaat g 21
<210> 4
   <211> 23
   <212> DNA
   <213> Artificial Sequence
<220>
   <223> BALF-5_P
<400> 4
   tgtacacgca cgagaaatgc gcc 23
<210> 5
   <211> 18
   <212> DNA
   <213> Artificial Sequence
<220>
   <223> ZEBRA_F
<400> 5
   ccaggcttgg gcacatct 18
<210> 6
   <211> 22
   <212> DNA
   <213> Artificial Sequence
<220>
   <223> ZEBRA_R
<400> 6
   cccatctaaa cgcctgattt tt 22
<210> 7
   <211> 28
   <212> DNA
   <213> Artificial Sequence
<220>
   <223> ZEBRA_P1
<400> 7
   cattttcaga tgatttggca gcagccac 28
<210> 8
<211> 16
   <212> DNA
   <213> Artificial Sequence
<220>
   <223> ZEBRA_P2
<400> 8
   cttcaacagg aggcgc 16

## Description

La présente invention concerne l'utilisation d'au moins un biomarqueur pour le pronostic ou le diagnostic *in vitro* d'épisodes lymphoprolifératifs associés au virus d'Epstein-Barr (EBV) chez un patient.

Le virus d'Epstein-Barr (EBV) est un virus de la famille des *Herpesviridae* qui infecte 90 à 95% de la population mondiale. EBV, virus à ADN double brin enveloppé, infecte préférentiellement les lymphocytes B mais est également capable d'infecter d'autres lignages cellulaires, notamment les lymphocytes T, les macrophages et les cellules épithéliales. EBV pénètre dans les lymphocytes B grâce à trois glycoprotéines essentielles (gp350/220, gp85 et gp42) lui permettant de lier le récepteur CD21 et les molécules du CMH (Complexe Majeur d'Histocompatibilité) de classe II présent à la surface des lymphocytes B quiescents. La primo-infection par EBV conduit à une activation du système immunitaire de l'hôte qui présente alors un profil sérologique standard, notamment par la production d'anticorps d'isotypes IgG et/ou IgM dirigés contre des antigènes viraux notamment EBNA (Epstein-Barr Nuclear antigen), EA (Early Antigen) ou VCA (Virus Capsid Antigen). Cette primo-infection, généralement asymptomatique, peut conduire à une mononucléose infectieuse qui se résout spontanément en quelques semaines chez un patient immunocompétent.

Une fois l'épisode de primo-infection résolu, EBV entre en phase de latence. Le génome d'EBV se circularise et est retrouvé sous forme épisomale dans le noyau des cellules infectées. Un nombre très restreint de gènes viraux est alors exprimé qui permettent au virus non seulement de maintenir son génome lors de la division de cellule hôte mais également d'échapper à la vigilance du système immunitaire. Parmi ces gènes, ceux codant pour les protéines nucléaires EBNA-1 (Epstein-Barr Nuclear Antigen-1) requis pour la réplication épisomale et la maintenance du génome viral, EBNA-2 (essentiel au processus d'immortalisation des lymphocytes B), EBNA-3 (impliquée dans la mise en place de la forme latente d'EBV), EBNA-5 (impliqué dans la transformation virale), et les protéines virales membranaires LMP-1 (Latent Membrane Protein-1) et LMP-2 jouent un rôle majeur. Les ARN viraux non codants EBER-1 (Epstein-Barr virus Encoded RNA-1) et EBER-2 exercent également une fonction clé en empêchant l'apoptose des cellules hôtes. Il existe en réalité plusieurs formes de latence qui correspondent à des profils distincts d'expression des gènes d'EBV précités. La forme 0, où aucun gène d'EBV n'est exprimé, correspond à la forme latente d'EBV retrouvée dans les lymphocytes B mémoires une fois la primo-infection résolue ; c'est la forme d'EBV la moins immunogène qui permet ainsi au virus d'échapper totalement au système immunitaire tout au long de la vie du patient. Une partie de ces lymphocytes B mémoires peut aussi contenir la forme 1 de latence (EBNA-1⁺/EBERs⁺) d'EBV. Les cellules infectées par la forme II-A (EBNA-1⁺/EBNA-2⁻/LMP-1⁺) sont retrouvées dans les centres germinaux des amygdales des porteurs sains. La forme de latence II-B (EBNA-1⁺/EBNA-2⁺/LMP-1⁻) correspond à celle retrouvée dans les cellules des amygdales des porteurs souffrant de mononucléose infectieuse. La forme III de latence (expression des six protéines EBNA et des trois protéines LMP) constitue la forme de latence la plus immunogène d'EBV et est généralement retrouvée dans les cellules transformées chez des patients immunodéficients. Cette forme latente d'EBV est également considérée comme la plus agressive car elle constitue la seule à pouvoir transformer efficacement des lymphocytes B *in vitro.*

L'implication des différentes formes latentes d'EBV a été rapportée dans de nombreuses pathologies de type néoplasique (lymphome de Burkitt, lymphome Hodgkinien et non-Hodgkinien, carcinome du nasopharynx et carcinome gastrique, syndromes lymphoprolifératifs) non seulement chez les patients immunodéprimés ou immunodéficients, mais également chez les patients possédant un système immunitaire intact (Carbone et al., The Oncologist 2008, 13 : 577-585). Toutefois, les sujets immunocompétents maintiennent une infection virale latente sous contrôle (réactivations périodiques d'EBV bien endiguées par le système immunitaire), alors que les sujets immunodéprimés ou immunodéficients souffrent de réactivations virales pouvant évoluer vers des lymphoproliférations malignes.

Les lymphoproliférations réprésentent une complication fréquente et le plus souvent létale chez les patients présentant un déficit immunitaire, qu'il soit d'origine génétique (syndrome de Wiskott-Aldrich, syndrome lymphoprolifératif lié à l'X), consécutif d'une infection par le virus de l'immunodéficience humaine (VIH) ou lié à la prise de traitements immunosuppresseurs chez les patients greffés. Pour cette dernière catégorie de patients, la stimulation antigénique chronique liée au greffon, l'effet oncogénique des traitements immunosuppresseurs, la diminution des réponses immunitaires et l'infection par l'EBV sont autant d'éléments qui contribuent au développement d'une telle lymphoprolifération.

Les pathologies lymphoprolifératives post-transplantation (PTLD, post transplant lymphoproliferative disease) constituent une complication souvent fatale chez les patients ayant reçu une transplantation d'organe solide ou une greffe de cellules souches hématopoïétiques. Chez les patients ayant reçu une greffe de moëlle osseuse, les PTLD se déclarent dans les 12 mois qui suivent l'implantation du greffon, c'est-à-dire avant que le système T ne soit complètement restauré. Les patients transplantés peuvent développer des PTLD la première année qui suit la transplantation (lorsque le traitement immunosuppresseur est le plus intense) mais également lors d'épisode d'immunosuppression prolongée chronique (Ethel Cesarman, Cancer Letters 2011, 305(2) : 163-172). Les PTLD se caractérisent par une prolifération non contrôlée des lymphocytes B infectés par EBV. Chez ces patients immunodéprimés, une hyporéactivité des lymphocytes T favorise une prolifération polyclonale puis monoclonale des lymphocytes B conduisant à la malignité.

Les pathologies précitées se développent consécutivement à une réactivation d'EBV. Cette réactivation correspond à l'activation de la réplication globale d'EBV faisant intervenir non seulement l'origine de réplication latente du virus (oriP, qui permet son maintient sous forme épisomale) mais également l'origine de réplication lytique (oriLyt) du virus. Alors que l'activation d'oriP est menée par les protéines d'origines cellulaires associées à EBNA-1, celle d'oriLyt requiert l'intervention de plusieurs protéines d'origine virale (Fixman *et al*., 1995, 69(5) : 2998-3006). L'activation d'oriLyt conduit à plusieurs cycles consécutifs de réplication de l'ADN viral. La réactivation d'EBV est caractérisée par une réplication forte faisant intervenir les deux origines de réplication du virus.

Lors de la réactivation d'EBV, une minorité de lymphocytes B infectés par EBV sous sa forme latente entrent en phase d'infection lytique. Au cours de cette phase, l'ensemble des protéines virales d'EBV sont produites permettant l'assemblage de virions complets qui lysent leurs cellules hôtes pour aller infecter les cellules voisines (cycle lytique). Cette transmission horizontale d'EBV a pour but d'accroître le pool de lymphocytes B infectés par le virus EBV. Dans un contexte lymphoprolifératif, les cellules infectées par la forme lytique d'EBV contribuent au processus de progression tumorale conduisant aux lymphomes (Hong et al., J. Virology 2005, 79(22) : 13993-14003 / Ma et al., J. Virology 2011, 85(1) : 165-177). Ces réactivations virales sont généralement sans conséquences chez les patients immunocompétents mais peuvent conduire à une lymphoprolifération maligne chez les patients immunodéficients ou immunodéprimés, pouvant aboutir à un lymphome.

Le déclenchement de la réactivation d'EBV est mal connu. Il a cependant été clairement démontré que la protéine virale ZEBRA (*Bam*HI Z EBV replication activator), également appelée Zta, joue un rôle très en amont de cette réactivation (George Miller, 1989 The Yale Journal of Biology and Medecine, 62 : 205-213, El-Guindy et al., PLos Pathogens 2010, 6(8) : e100105). La protéine ZEBRA, codée par le gène BZLF-1, est un facteur de transcription de la famille des leucine-zipper basiques qui joue un rôle essentiel dans la transcription des gènes viraux mais également dans l'activation de la réplication du virus. La protéine ZEBRA est responsable du passage d'EBV de sa phase latente à sa phase lytique. La protéine comporte trois domaines fonctionnels distincts: un domaine transactivateur, un domaine de liaison à l'ADN et un domaine de dimérisation (Petosa et al., Molecular Cell 2006, 21(4) : 565-572). Ce dernier domaine, appelé domaine ZANK (ZEBRA ANKryn-like Region) permet non seulement à la protéine ZEBRA de s'homodimériser mais également de lier les protéines p53 et NF-kB (Dreyfus et al. Virology Journal 2011, 8:422). L'interaction de la protéine ZEBRA avec les protéines p53 et NF-kB pourrait jouer un rôle dans l'immunopathologie et la carcinogénèse virale des lymphocytes B induite par l'EBV et des autres lignages cellulaires infectés transitoirement par l'EBV.

La surexpression de la protéine ZEBRA dans des lignées cellulaires de lymphocytes conduit à la formation de dimères protéiques ZEBRA-ZAP ou ZEBRA-RACK1 capables de moduler respectivement l'activité trans-activatrice de la protéine ZEBRA ou de perturber la transduction du signal dépendant de la protéine kinase C (Katz et al., PNAS, 1992, 89, 378-382 ; Tardif et al., JBC, 2002, 277(27), 24148-24154). Toutefois, ces dimères protéiques ont une localisation purement intracellulaire, soit dans le cytosol soit dans le noyau des cellules surexprimant la protéine ZEBRA ; ces dimères sont absents du milieu de culture. L'accès à ces dimères n'est possible qu'après une lyse des cellules, rendant leur dosage par des anticorps indirect.

L'étude de pathologies en lien avec la réactivation d'EBV a permis l'identification de marqueurs biologiques permettant le pronostic ou le diagnostic de lymphoproliférations chez des patients. Parmi ces marqueurs, ZEBRA constitue un marqueur pertinent de la réactivation d'EBV.

Sur le plan biologique, la réactivation d'EBV a été caractérisée par la titration des anticorps anti-ZEBRA chez des patients porteurs de la maladie de Hodgkin (Drouet et al., J. Med. Virol. 1999, 57(4):383-9). La détection des anticorps anti-ZEBRA constitue également un marqueur pronostique chez des patients souffrant de carcinome du nasopharynx (Tedeschi et al., Clinical and Vaccine Immunology 2007, 14(4) : 435-441 / Dardari et al., J. Clin. Virol. 2008, 41(2) : 96-103). La détection de l'antigène ZEBRA a également servi de marqueur diagnostique du PTLD chez des patients greffés. Toutefois, sa mesure nécessite la réalisation de biopsies sur lesquelles sont pratiquées des techniques d'immunohistochimie (Oertel et al., B. J. Haematology 2002, 118 :1120-1123). Le recours à de telles techniques ne peut être envisagé que dans le cas ou la lymphoprolifération est localisée à un endroit précis de l'organisme et non pas diffuse dans des tissus distincts.

Les techniques de biologie moléculaire ont permis l'émergence de tests commerciaux de diagnostic *in vitro* de la mesure de la charge virale d'EBV. Cette mesure ne constitue pas à elle seule un marqueur diagnostique du PTLD mais constitue un indice diagnostique renseignant le praticien sur la nécessité d'utiliser ou non des antiviraux. Cependant, les techniques utilisée sont peu standardisées et varient d'un centre d'analyse à l'autre (Preiksaitis et al., Am. J. Transplant. 2009, 9(2) : 269-279). De plus, les travaux de Oertel montrent que la mesure de la charge virale n'est pas un bon marqueur prédictif des PTLD (Oertel et al., 2006, Ann. Hematol., 85(7) : 478-484).

Il existe par conséquent un réel besoin d'identification de marqueurs diagnostiques de la réactivation d'EBV qui lorsqu'on les associe à d'autres marqueurs biologiques déjà connus permettent le pronostic ou le diagnostic d'épisodes lymphoprolifératifs, qu'il s'agisse d'un premier épisode ou bien encore d'une rechute.

Un des buts de la présente invention est de fournir un biomarqueur qui soit à la fois un marqueur précoce de la réactivation d'EBV et qui, lorsqu'il est associé à d'autres biomarqueurs, puisse permettre le pronostic ou le diagnostic d'une lymphoprolifération.

Un autre but de l'invention est de fournir un procédé de mise en évidence dudit biomarqueur qui soit facile à mettre en oeuvre, non invasif et dont le coût ne constitue pas un facteur limitant d'utilisation.

Encore un autre but de l'invention est de fournir des trousses diagnostiques utilisables pour le pronostic ou le diagnostic d'épisodes lymphoprolifératifs chez des patients séropositifs pour EBV.

Enfin, l'invention a pour but de fournir un biomarqueur qui soit utilisable tant chez les patients immunodéprimés (ou immunodéficients) qu'immunocompétents.

La présente divulgation concerne un complexe protéique isolé de son milieu naturel et comprenant la protéine ZEBRA de séquence SEQ ID NO: 1, ledit complexe protéique isolé possédant les propriétés suivantes :
- il est plus stable que la protéine ZEBRA, notamment il est plus résistant à l'action des protéases que la protéine ZEBRA,
- il est capable de lier spécifiquement les anticorps monoclonaux AZ125 et AZ130,
- il est soluble dans un liquide biologique, et notamment un liquide biologique choisi dans le groupe constitué par le sang et le sérum.

La présente divulgation concerne également un complexe protéique isolé de son milieu naturel et comprenant la protéine ZEBRA de séquence SEQ ID NO: 1, ledit complexe protéique isolé possédant les propriétés suivantes :
- il est plus stable que la protéine ZEBRA, notamment il est plus résistant à l'action des protéases que la protéine ZEBRA,
- il est capable de lier spécifiquement les anticorps monoclonaux AZ125 et AZ130,
- il est soluble dans un liquide biologique issu de patients séropositifs pour EBV, et notamment un liquide biologique choisi dans le groupe constitué par le sang et le sérum,
- il est sous une forme circulante dans ledit liquide biologique issu de patients séropositifs pour EBV,
- il est dosable directement dans ledit liquide biologique issu de patients séropositifs pour EBV.

Les inventeurs ont mis en évidence pour la première fois l'existence d'une forme circulante de la protéine ZEBRA sous une forme protégée (complexe protéique). De plus, les inventeurs ont montré que l'antigène ZEBRA sous sa forme circulante protégée pouvait être dosé directement à partir d'échantillons de sang total des patients séropositifs pour EBV.

Par « complexe protéique », on désigne l'association physique d'au moins deux protéines, l'une d'elle étant impérativement la protéine ZEBRA. On désigne également l'association physique entre la protéine ZEBRA et un acide nucléique. Dans la présente invention, la protéine ZEBRA est la protéine virale native. Par protéine virale native, on désigne la séquence protéique codée par une portion d'acides nucléiques comprise dans le génome d'EBV. L'association physique entre la protéine ZEBRA et une autre protéine ou la protéine ZEBRA et un acide nucléique repose sur des liaisons non covalentes entre les chaines polypeptidiques desdites protéines ou entre la chaine polypeptidique de la protéine ZEBRA et des séquences spécifiques de l'acide nucléique. Cette association permettrait non seulement de protéger la protéine ZEBRA de l'action des protéases mais également de laisser l'accessibilité aux fragments antigéniques de la protéine ZEBRA reconnus par les anticorps monoclonaux AZ125 et AZ130.

Par « isolé de son milieu naturel », on désigne un complexe protéique qui a été substantiellement séparé ou purifié des autres composants du liquide biologique, c'est-à-dire des autres constituants de nature protéique, lipidique ou glucidique du sang ou du sérum. Le terme « isolé de son milieu naturel » n'englobe pas un complexe protéique comprenant une forme recombinante de la protéine ZEBRA. A titre d'exemples et sans que cela ne restreigne l'objet de la présente invention, la purification du complexe protéique comprenant la protéine ZEBRA de la présente invention peut être réalisée à l'aide d'une des techniques suivantes:
(i) chromatographie par échange d'ions : dialyse de l'échantillon biologique, notamment du plasma contre un tampon alcalin Tris 0,1M pH8, puis passage sur colonne DEAE cellulose. L'élution du complexe protéique comprenant la protéine ZEBRA se fait par un gradient discontinu de NaCl (0,1M- 0,15 M-0,25M-0,5M-0,75M-1M). Les fractions collectées sont analysées par électrophorèse SDS-PAGE et coloration en bleu de Coomassie ; ou bien
(ii) chromatographie gel-filtration SUPERDEX S200. Cette technique qui ne nécessite pas d'élution peut éventuellement être associée avec la méthode décrite en (i) pour augmenter le degré de pureté de ZEBRA ; ou bien
(iii) chromatographie d'affinité : colonne de protéine A ou protéine G sur lesquelles sont fixés les anticorps monoclonaux anti-ZEBRA purifiés AZ125 et/ou AZ130. La préparation dialysée est déposée sur la colonne, puis soumis à l'élution à l'aide de l'acide citrique 0,1M pH3. L'éluat est analysé en SDS-PAGE et en spectro de masse et MALLS (multiangle laser light scattering) pour définir la masse moléculaire. Une variante de cette technique consiste à utiliser des billes de sépharose activées sur lesquelles sont fixés les anticorps AZ125 et/ou AZ130.

Par « protéine ZEBRA de séquence SEQ ID NO : 1 », on désigne la protéine de 245 acides aminés comprenant ou consistant en la SEQ ID NO : 1. On désigne également les variants de la protéine ZEBRA c'est-à-dire les protéines homologues de celle ayant la SEQ ID NO : 1, lesdites protéines présentant au moins 50 % d'identité avec la région protéique contenant les acides aminés de 176 à 194 de la SEQ ID NO : 1, ladite région correspondant au domaine de liaison à l'ADN de la protéine ZEBRA.

Par « la protéine ZEBRA », on désigne la protéine ZEBRA seule, sous sa forme native ou recombinante, c'est-à-dire non liée à au moins une autre protéine ou un autre constituant. Par protéine recombinante, on désigne une protéine produite par une cellule hôte dans laquelle un vecteur d'expression comportant la séquence codante de la protéine d'intérêt a été introduit. Les techniques de clonage et de production de protéines recombinantes sont bien connues de l'homme de l'art. Dans le cas de la présente invention, par protéine recombinante ZEBRA, on désigne la protéine comprenant ou consistant en la SEQ ID NO : 1, et également les protéines homologues de celle ayant la SEQ ID NO : 1, lesdites protéines présentant au moins 50 % d'identité avec la région protéique contenant les acides aminés de 176 à 194 de la SEQ ID NO : 1, ladite région correspondant au domaine de liaison à l'ADN de la protéine ZEBRA.

Par « plus stable », on désigne la capacité du complexe comprenant la protéine ZEBRA à conserver ses propriétés fonctionnelles dans le temps. Selon la présente invention, la durée de demi-vie du complexe comprenant la protéine ZEBRA est supérieure à celle de la protéine ZEBRA non complexée. La protéine ZEBRA lorsqu'elle se trouve dans le complexe protéique conserve un état structuré. Inversement, la protéine ZEBRA non complexée voit sa structure tertiaire plus rapidement déstabilisée. Cette stabilité peut être évaluée après purification du complexe protéique par chromotographie d'affinité sur une colonne contenant un anticorps anti-ZEBRA. Les techniques d'analyse physico-chimiques sont bien connues de l'homme de l'art (Flamand et al., 1999, J. Virol., 73(7) : 6104-6110), et reposent notamment sur des expériences de dissociation du complexe isolé et purifié à l'aide de détergent, l'analyse par diffraction des rayons X ou par spectroscopie RMN (Résonnance Magnétique Nucléaire), ou tout autre technique bien connue de l'homme de l'art.

Par « plus résistant », on désigne la capacité de la protéine ZEBRA dans le complexe protéique à résister aux différentes voies de dégradation des protéines dans les cellules eucaryotes. Il s'agit notamment des protéases, du lysosome ou encore de la voie de l'ubiquitine-protéasome.

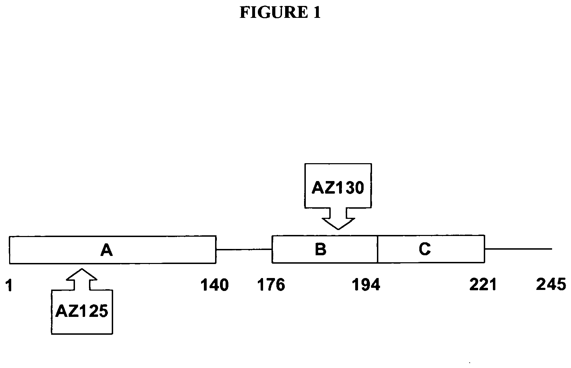

Par « capable de lier spécifiquement les anticorps monoclonaux AZ125 et AZ130 », on désigne des anticorps qui lient substantiellement une cible unique. Dans le cas de la présente invention, les anticorps monoclonaux murins AZ125 et AZ130 sont spécifiquement dirigés contre les fragments N et C terminaux de la protéine ZEBRA (figure 1). Le complexe protéique comprenant la protéine ZEBRA est capable de se lier à ces anticorps et à d'autres anticorps monoclonaux ou polyclonaux dirigés spécifiquement contre des épitopes immunogènes de la protéine ZEBRA pour former un complexe immun mais ne peut pas lier des anticorps dirigés contre des antigènes n'ayant aucune structure commune avec ce complexe.

L'anticorps monoclonal AZ125 est disponible auprès de la société Argene SA (Verniolle - France) sous le nom commercial anti EBV-ZEBRA purifié, sous la référence 11-007. AZ125 est dirigé contre le peptide 1-140 de la protéine ZEBRA, c'est-à-dire contre l'exon 1 qui code pour le domaine transactivateur de la protéine ZEBRA.

L'hybridome sécrétant l'anticorps monoclonal AZ130 a été déposé auprès de la CNCM (Collection Nationale de Culture de Microorganismes, Institut Pasteur, 25 rue du Docteur Roux, 75724 Paris Cedex 15, France) sous le traité de Budapest le 17 novembre 2011, sous la référence CNCM I-4554, ledit hybridome et ledit anticorps AZ130 font également partie de la présente invention.

AZ130 est dirigé contre le peptide 176-194 de la protéine ZEBRA, c'est-à-dire contre l'exon 2 qui code pour le domaine de liaison à l'ADN de la protéine ZEBRA.

La présente invention concerne également un anticorps qui lie spécifiquement le fragment C terminal de la protéine ZEBRA de séquence SEQ ID NO : 1.
Par « fragment C terminal », on désigne le peptide de 39 acides aminés dont la séquence inclut les acides aminés 157 à 195 de la protéine ZEBRA de SEQ ID NO : 1.

La présente invention concerne également un hybridome déposé à la CNCM (Collection Nationale de Culture de Microorganismes, Institut Pasteur, 25 rue du Docteur Roux, 75724 Paris Cedex 15, France) sous le traité de Budapest le 17 novembre 2011, sous la référence CNCM I-4554, capable de sécréter l'anticorps AZ130.

La présente invention concerne également un anticorps AZ130 qui lie spécifiquement le fragment C terminal de la protéine ZEBRA de séquence SEQ ID NO: 1, l'anticorps AZ130 étant l'anticorps sécrété par l'hybridome déposé à la CNCM (Collection Nationale de Culture de Microorganismes, Institut Pasteur, 25 rue du Docteur Roux, 75724 Paris Cedex 15, France) sous le traité de Budapest le 17 novembre 2011, sous la référence CNCM I-4554.

Par « soluble dans un liquide biologique », on désigne la capacité du complexe comprenant la protéine ZEBRA à ne pas former d'agrégat, ni à précipiter dans un liquide biologique à pH physiologique. Une étude de la dispersion du complexe protéique dans un liquide biologique peut être réalisée à partir du complexe protéique isolé et purifié, notamment par la diffraction des rayons X aux petits angles ou par toute autre technique bien connue de l'homme de l'art.

Par « sous une forme circulante », on désigne le complexe protéique isolé de son milieu naturel et comprenant la protéine ZEBRA qui se trouve à l'extérieur des cellules, c'est-à-dire qu'il circule librement dans les fluides biologiques. La « forme circulante » signifie que le complexe n'est localisé dans aucun des compartiments intra-cellulaires mais qu'il se trouve dans le milieu extracellulaire.

Par « dosable directement », on désigne la possibilité de former un complexe immun entre le complexe protéique isolé de son milieu naturel et comprenant la protéine ZEBRA et les anticorps AZ125 et/ou AZ130 sans qu'il soit nécessaire de procéder à une lyse cellulaire qui permettrait le relargage dudit complexe dans le milieu extracellulaire comme cela est le cas pour les dimères protéiques connus de l'art antérieur qui comprennent la protéine ZEBRA.

La présente divulgation concerne également un complexe protéique comprenant la protéine ZEBRA et possédant les propriétés susmentionnées, susceptible d'être obtenu par purification à partir du milieu extracellulaire d'une culture de cellules infectées par le virus d'Epstein-Barr (EBV).

Les cellules de la lignée Akata ont été isolées à partir d'un patient souffrant d'un lymphome de Burkitt. Cette lignée cellulaire, qui a intégré EBV sous sa forme épisomale, est l'une des lignées les plus importantes en termes de capacité de production d'EBV. En conditions de culture standard, les cellules de la lignée Akata demeurent à l'état latent. Le dosage de l'antigène ZEBRA est alors inférieur à 0,15 µg/ml de surnageant de culture. Ceci correspond à une phase de réplication faible pour EBV au cours de laquelle seule oriP est activée. Cette phase de réplication faible ne permet pas la détection d'EBV. Lorsqu'elles sont activées à l'aide d'anticorps de type anti-IgG humaines, les cellules entrent en phase lytique et permettent ainsi le relargage de particules virales d'EBV dans le milieu extracellulaire. Le dosage de l'antigène ZEBRA est alors d'environ 2,67 µg/ml de surnageant de culture. Ceci correspond à une phase de réplication forte pour EBV au cours de laquelle les deux origines de réplication virale oriP et oriLyt sont activées.

Ainsi, selon un mode de réalisation particulier, ladite culture est la culture d'une lignée cellulaire appartenant au groupe constitué par toutes lignées cellulaires appropriées pour l'infection par la forme réplicative d'EBV, et notamment la lignée Akata.

Par « forme réplicative », on désigne la forme d'EBV où les deux origines de réplications oriP et oriLyt sont activées. Il s'agit de la forme virale conduisant à la production de particules virales complètes capables d'infecter des cellules en culture. Cette forme réplicative est caractéristique de la réactivation d'EBV.

Les inventeurs ont mis en évidence que les taux de la protéine ZEBRA circulante varient en fonction de l'état de réactivation du virus EBV et que cette protéine est retrouvée à un taux élevé dans le sang total de patients susceptibles de développer une lymphoprolifération.

La présente divulgation concerne également un procédé de mise en évidence, dans un échantillon biologique issu d'un patient séropositif pour EBV, d'un complexe protéique comprenant la protéine ZEBRA et possédant les propriétés susmentionnées, ledit procédé comprenant les étapes :
(a) d'obtention d'un échantillon biologique issu d'un patient séropositif pour EBV,
(b) de détermination de la présence d'un complexe immun, ledit complexe immun étant constitué du susdit complexe protéique lié à l'un des ligands suivants : anticorps AZ125, anticorps AZ130 ou anticorps AZ125 et AZ130 pris en combinaison, par comparaison avec un échantillon biologique issu d'un patient séronégatif pour EBV.

La présente divulgation concerne également, un procédé de mise en évidence, dans un échantillon biologique notamment choisi parmi le groupe constitué par le sang et le sérum issu d'un patient séropositif pour EBV, d'un complexe protéique comprenant la protéine ZEBRA et possédant les propriétés susmentionnées, comprend l'étape suivante :
- détermination de la présence d'un complexe immun dans un échantillon biologique préalablement prélevé d'un patient séropositif pour EBV, ledit complexe immun étant constitué du susdit complexe protéique lié à l'un des ligands suivants : anticorps AZ125, anticorps AZ130 ou anticorps AZ125 et AZ130 pris en combinaison, par comparaison avec un échantillon biologique préalablement prélevé d'un patient séronégatif pour EBV.

La présente invention concerne une méthode de pronostic ou de diagnostic *in vitro* et/ou *ex-vivo* d'une lymphoprolifération associée à la forme réplicative d'EBV ou plus généralement d'une tumeur associée à EBV, en particulier le cancer du nasopharynx, ladite méthode nécessitant la mise en évidence de la présence de la protéine ZEBRA de séquence SEQ ID NO: 1, dans un échantillon biologique choisi parmi le groupe constitué par le sang et le sérum, d'un patient séropositif pour EBV,
ladite mise en évidence de la protéine ZEBRA de séquence SEQ ID NO: 1 comprenant l'étape suivante :
- détermination de la présence d'un complexe immun dans un échantillon biologique préalablement prélevé d'un patient séropositif pour EBV, ledit complexe immun étant constitué de la susdite protéine liée aux ligands suivants : anticorps monoclonal dirigé contre le peptide 1-140 de la protéine ZEBRA de SEQ ID NO : 1 AZ125 et anticorps monoclonal AZ130 pris en combinaison, l'anticorps AZ130 étant l'anticorps sécrété par l'hybridome déposé à la CNCM (Collection Nationale de Culture de Microorganismes, Institut Pasteur, 25 rue du Docteur Roux, 75724 Paris Cedex 15, France) sous le traité de Budapest le 17 novembre 2011, sous la référence CNCM I-4554,
ladite quantité de protéine ZEBRA de séquence SEQ ID NO: 1 pronostique ou diagnostique d'une lymphoprolifération étant notamment égale ou supérieure à 0,15 microgrammes/ml d'échantillon biologique.

Par « patient séropositif pour EBV », on désigne un patient qui a été infecté par le virus EBV et dont le profil sérologique montre qu'il ne s'agit pas d'une primo-infection mais bien d'une infection ancienne. En d'autres termes, le patient ne souffre pas de mononucléose infectieuse mais est porteur du virus EBV principalement sous sa forme latente. Le profil sérologique est basé sur la détection d'anticorps anti-protéines d'EBV bien connue de l'homme de l'art. Il s'agit notamment des IgG et IgM anti-VCA, d'IgG anti-EA ou d'IgG anti-EBNA. Le profil sérologique peut être classique ou atypique en fonction de l'état du système immunitaire du patient. Par « patient », on désigne un mammifère, notamment un être humain ou un animal.

Par « obtention d'un échantillon biologique », on désigne l'étape de prélèvement dudit échantillon biologique réalisé directement sur le patient séropositif pour EBV.

Par « complexe immun », on désigne l'interaction physique entre un antigène et un anticorps spécifiquement dirigé contre cet antigène. Dans la présente invention, cette interaction est réalisée *in vitro* entre le complexe protéique comprenant la protéine ZEBRA et les anticorps monoclonaux murins AZ125 et/ou AZ130, spécifiquement dirigés contre les fragments N et C terminaux de la protéine ZEBRA.

Selon un mode de réalisation particulier, le procédé de l'invention peut être mis en oeuvre sur un échantillon biologique choisi parmi le groupe constitué par le sang et le sérum.

Selon un mode de réalisation plus particulier, dans le susdit procédé de la présente divulgation, la liaison du complexe protéique à l'un des ligands est détectée à l'aide d'un test de type immunoessai.

Selon un mode de réalisation encore plus particulier, dans le susdit procédé de la présente divulgation, l'immunoessai est de type ELISA sandwich dans lequel l'anticorps AZ125 est adsorbé sur une plaque appropriée et l'anticorps AZ130 est utilisé pour la détection de la fixation du complexe protéique à l'anticorps AZ125 adsorbé.

Selon un mode de réalisation particulier, dans la susdite méthode selon la présente invention, la liaison de la protéine ZEBRA de séquence SEQ ID NO : 1 à l'un des ligands est détectée à l'aide d'un test de type immunoessai, notamment de type ELISA sandwich dans lequel l'anticorps AZ125 est adsorbé sur une plaque appropriée et l'anticorps AZ130 est utilisé pour la détection de la fixation de la protéine ZEBRA de séquence SEQ ID NO : 1 à l'anticorps AZ125 adsorbé.

La formation du complexe immun peut avoir lieu entre le complexe protéique comprenant la protéine ZEBRA et l'un seulement des deux anticorps monoclonaux murins AZ125 ou AZ130.

La formation du complexe immun peut avoir lieu entre le complexe protéique comprenant la protéine ZEBRA et les deux anticorps monoclonaux murins AZ125 et AZ130.

La mise en oeuvre de test d'immunodétection est bien connue de l'homme de l'art. La détection et le dosage d'antigène peut être réalisée par immunocapture, en sandwich ou en compétition. Les moyens de détection sont généralement des anticorps hétérologues anti-humains couplés soit à une enzyme capable de catalyser une réaction colorée soit à un marqueur fluorescent ou luminescent.

La détection du taux de la protéine ZEBRA chez les patients peut être associée à l'un au moins des autres marqueurs biologiques pertinents en termes d'infection du patient par le virus EBV.

Selon un autre mode de réalisation, le procédé de l'invention comprend en outre la détection des anticorps anti-ZEBRA.

Par « anticorps anti-ZEBRA », on désigne tous les anticorps synthétisés par le système immunitaire d'un patient séropositif pour EBV spécifiquement dirigés contre la protéine virale ZEBRA. La détection de ces anticorps peut être effectuée en utilisant une forme recombinante de la protéine ZEBRA ou bien des peptides naturels ou synthétiques dérivés directement de la séquence de la protéine ZEBRA.

La détection des anticorps anti-ZEBRA peut être effectuée sur le même échantillon que celui ayant servi à la détection du complexe protéique comprenant la protéine ZEBRA, ou sur un autre échantillon (de même nature ou de nature différente, notamment une biopsie) prélevé le même jour que celui ayant servi à la détection du complexe protéique comprenant la protéine ZEBRA.

La détection du complexe protéique comprenant la protéine ZEBRA peut être associée à la détection des anticorps anti-ZEBRA.

Selon un autre mode de réalisation, le procédé de l'invention comprend en outre la mesure de la charge virale d'EBV dans un échantillon biologique du patient.

Par « charge virale », on désigne la détection du génome d'EVB chez un patient séropositif pour ce virus. La quantification de l'acide nucléique d'EBV peut être absolue ou relative. L'unité de quantification peut être exprimée en copies/ml, copies/µg d'ADN cellulaire qui est équivalent à copies/150 000 cellules (1µg représente 150 000 cellules) ou en UI (Unité Internationale) / ml (First WHO International Standard for EBV, NIBSC 09/260).

La mesure de la charge virale peut être effectuée sur le même échantillon que celui ayant servi à la détection du complexe protéique comprenant la protéine ZEBRA, ou sur un autre échantillon (de même nature ou de nature différente, notamment une biopsie) prélevé le même jour que celui ayant servi à la détection du complexe protéique comprenant la protéine ZEBRA.

La détection du complexe protéique comprenant la protéine ZEBRA peut être associée à la mesure de la charge virale.

La détection du complexe protéique comprenant la protéine ZEBRA peut être associée à la mesure de la charge virale et à la détection des anticorps anti-ZEBRA.

Selon un mode de réalisation particulier, dans le procédé de l'invention la charge virale est détectée par une technique de type PCR.

Par « PCR », on désigne la réaction de polymérisation en chaîne qui permet l'amplification d'un acide nucléique *in vitro* en un grand nombre de copies identiques entre elles et identiques à l'acide nucléique ayant servi de matrice d'amplification.

Il existe dans l'art antérieur un couple d'amorces et de sonde permettant la détection du gène BALF-5 codant pour l'ADN polymérase d'EBV (Kimura et al., J Clin Microbiol. 1999 Jan;37(1):132-6). Les séquences de ces amorces et de cette sonde correspondent respectivement aux séquences SEQ ID NO: 2 pour l'amorce sens, SEQ ID NO: 3 pour l'amorce anti-sens et SEQ ID NO: 4 pour la sonde. Les séquences nucléotidiques figurent dans le tableau 2 de l'exemple 2 de la présente demande.

Par ailleurs, les inventeurs ont également choisi un couple d'amorces et deux sondes permettant la détection du gène BZLF-1 codant pour la protéine virale ZEBRA d'EBV. La séquence du génome d'EBV souche B95-8 ayant servi aux choix des amorces et des sondes est accessible dans les bases de données sous la référence GenBank V01555.2. Les séquences de ces amorces et de cette sonde correspondent respectivement aux séquences SEQ ID NO: 5 pour l'amorce sens, SEQ ID NO: 6 pour l'amorce anti-sens et SEQ ID NO: 7 ou SEQ ID NO: 8 pour la sonde. Les séquences nucléotidiques figurent dans le tableau 2 de l'exemple 2 de la présente demande.

Selon un mode de réalisation particulier, dans le procédé de l'invention une PCR est mise en oeuvre en utilisant des amorces et les sondes dont les séquences en acides nucléiques sont choisies dans le groupe constitué par : SEQ ID NO: 2, SEQ ID NO:3, SEQ ID NO:4.

Selon un autre mode de réalisation particulier, dans le procédé de l'invention une PCR est mise en oeuvre en utilisant des amorces et les sondes dont les séquences en acides nucléiques sont choisies dans le groupe constitué par : SEQ ID NO: 5, SEQ ID NO: 6, SEQ ID NO: 7, SEQ ID NO: 8.

Selon un encore un autre mode de réalisation particulier, dans le procédé de l'invention une PCR est mise en oeuvre en utilisant des amorces et les sondes dont les séquences en acides nucléiques sont choisies dans le groupe constitué par : SEQ ID NO: 2, SEQ ID NO: 3, SEQ ID NO: 4, SEQ ID NO: 5, SEQ ID NO: 6, SEQ ID NO: 7, SEQ ID NO: 8.
Selon un encore un autre mode de réalisation particulier, dans le procédé de l'invention, la mise en évidence de la protéine ZEBRA de séquence SEQ ID NO: 1 comprend en outre la mesure de la charge virale d'EBV, notamment par une technique de type PCR, notamment en utilisant des amorces et des sondes dont les séquences en acides nucléiques sont choisies dans le groupe constitué par : SEQ ID NO: 2, SEQ ID NO: 3, SEQ ID NO: 4, SEQ ID NO: 5, SEQ ID NO: 6, SEQ ID NO: 7, SEQ ID NO: 8.

Par « amorces et sondes », on désigne des séquences d'acide nucléique variant de 8 à 30 oligonucléotides. Les amorces sont capables de s'hybrider à une séquence d'ADN qui leur est complémentaire pour former un hybride ADN-ADN. L'ADN polymérase peut alors étendre le brin d'ADN en aval du primer. Une sonde est une séquence d'acide nucléique liée à un moyen de détection, notamment un fluorophore. Les techniques de design d'amorces et de sondes sont des techniques de biologie moléculaire bien connues de l'homme de l'art (Molecular Cloning : A laboratory Manual, CHSL, New York, 1989 / Current Protocols in Molecular Biology, John Wiley & Son, New York, 1998).

Les inventeurs ont montré dans des lignées lymphoblastoïdes infectées par EBV qu'au moment de la réactivation du virus, la protéine ZEBRA est capable d'activer le promoteur du gène codant pour l'IL-10 ayant pour conséquence l'augmentation de l'expression de cette cytokine et son relargage dans le milieu extracellulaire. L'IL-10 possède une activité immunomodulatrice et favorise la prolifération des lymphocytes B. Il apparait par conséquent pertinent de suivre l'évolution de la concentration de cette cytokine chez les patients.

Selon un autre mode de réalisation, le procédé de l'invention comprend en outre le dosage de l'interleukine-10 (IL-10) dans un échantillon biologique du patient.

Par « interleukine 10 », on désigne la cytokine capable d'inhiber la production d'interféron-γ et la synthèse de cytokine pro-inflammatoires comme l'IL-1, l'IL-6 et le TNF-α (Tumor Necrosis Factor-alpha). Le dosage de l'IL-10 peut être effectué à l'aide d'anticorps spécifiquement dirigé contre cette cytokine.

Le dosage de l'IL-10 peut être effectué sur le même échantillon que celui ayant servi à la détection du complexe protéique comprenant la protéine ZEBRA, ou sur un autre échantillon (de même nature ou de nature différente, notamment une biopsie) prélevé le même jour que celui ayant servi à la détection du complexe protéique comprenant la protéine ZEBRA.

La détection du complexe protéique comprenant la protéine ZEBRA peut être associée au dosage de l'IL-10.

La détection du complexe protéique comprenant la protéine ZEBRA peut être associée au dosage de l'IL-10 et à la détection des anticorps anti-ZEBRA.

La détection du complexe protéique comprenant la protéine ZEBRA peut être associée au dosage de l'IL-10 et à la mesure de la charge virale.

La détection du complexe protéique comprenant la protéine ZEBRA peut être associée au dosage de l'IL-10, à la détection des anticorps anti-ZEBRA et à la mesure de la charge virale.

La présente divulgation concerne également l'utilisation de la mise en évidence de la présence d'un complexe protéique isolé de son milieu naturel et comprenant la protéine ZEBRA de séquence SEQ ID NO: 1, ledit complexe protéique isolé possédant les propriétés suivantes :
- il est plus stable que la protéine ZEBRA, notamment il est plus résistant à l'action des protéases que la protéine ZEBRA,
- il est capable de lier spécifiquement les anticorps monoclonaux AZ125 et AZ130,
- il est soluble dans un liquide biologique, et notamment un liquide biologique choisi dans le groupe constitué par le sang et le sérum,
dans un échantillon biologique d'un patient séropositif pour EBV, pour le pronostic ou le diagnostic d'une lymphoprolifération associée à la forme réplicative d'EBV ou plus généralement d'une tumeur associée à EBV, en particulier le cancer du nasopharynx.

La présente divulgation concerne également une méthode de pronostic ou de diagnostic *in vitro* et/ou *ex-vivo* d'une lymphoprolifération associée à la forme réplicative d'EBV ou plus généralement d'une tumeur associée à EBV, en particulier le cancer du nasopharynx, ladite méthode nécessitant la mise en évidence de la présence d'un complexe protéique comprenant la protéine ZEBRA et possédant les propriétés susmentionnées, dans un échantillon biologique d'un patient séropositif pour EBV.
La présente invention concerne également une méthode de pronostic ou de diagnostic *in vitro* et/ou *ex-vivo* d'une lymphoprolifération associée à la forme réplicative d'EBV ou plus généralement d'une tumeur associée à EBV, en particulier le cancer du nasopharynx, ladite méthode nécessitant la mise en évidence de la présence de la protéine ZEBRA dans un échantillon biologique d'un patient séropositif pour EBV.

Cette lymphoprolifération est une conséquence directe de la réactivation d'EBV caractérisée par l'activation des deux origines de réplication virales oriP et oriLyt. Ce processus conduit à une réplication forte d'EBV.

Par « lymphoprolifération », on désigne une multiplication non contrôlée de lymphocytes anormaux, qu'ils soient issus de la lignée B ou de la lignée T. Par non contrôlée, on désigne l'incapacité du système immunitaire à détruire les lymphocytes anormaux surnuméraires. Dans le cas du lignage B, cette multiplication peut être la conséquence de l'immortalisation des lymphocytes B par le virus EBV.

Selon un mode de réalisation particulier, dans l'utilisation de l'invention, la quantité de complexe protéique pronostique ou diagnostique d'une lymphoprolifération est environ égale ou supérieure à 0,15 microgrammes/ml d'échantillon biologique.

Selon un autre mode de réalisation particulier, dans la méthode de pronostic ou de diagnostic *in vitro* et/ou *ex-vivo* d'une lymphoprolifération associée à la forme réplicative d'EBV ou plus généralement d'une tumeur associée à EBV, en particulier le cancer du nasopharynx, la quantité de complexe protéique pronostique ou diagnostique d'une lymphoprolifération est environ égale ou supérieure à 0,15 microgrammes/ml d'échantillon biologique.

Selon un mode de réalisation plus particulier, dans l'utilisation de l'invention, le pronostic intervient dans un intervalle de temps variant de 1 à 90 jours avant la confirmation clinique d'une lymphoprolifération.

Selon un mode de réalisation particulier, dans l'utilisation de l'invention, le statut immunologique du patient appartient au groupe : patient immunocompétent, patient immunodéprimé ou patient immunodéficient.

Par «patient immunocompétent», on désigne un patient dont le système immunitaire remplit pleinement ses fonctions de défense de l'organisme vis-à-vis du « non-soi », c'est-à-dire des infections causées par les micro-organismes, notamment par les bactéries, virus, levures et champignons. Un patient immunocompétent au sens de la présente invention, séropositif pour EBV, présente un profil sérologique classique vis-à-vis des marqueurs viraux EBNA, VCA et EA : IgG anti-VCA positif/IgM anti-VCA négatif/IgA anti-VCA négatif/ IgG anti-EA négatif/ IgG anti-EBNA positif.

Par «patient immunodéprimé», on désigne un patient dont le système immunitaire fonctionne de manière imparfaite. C'est notamment le cas d'un patient en attente de greffe ou greffé ayant reçu un traitement immunosuppresseur. Dans ce cas, c'est un traitement chimique qui est à la base du dysfonctionnement du système immunitaire.

Par «patient immunodéficient», on désigne un patient dont le système immunitaire fonctionne de manière imparfaite. C'est notamment le cas d'un patient présentant une pathologie de type syndrome de Wiskott-Aldrich ou syndrome lymphoprolifératif lié à l'X ou souffrant de toute autre pathologie impliquant le système immunitaire. C'est également le cas d'un patient infecté par un virus perturbant le système immunitaire, notamment par les rétrovirus de l'immunodéficience humaine (VIH), le virus T lymphotropique humain (HTLV) ou le virus xénotropique lié à MuLV (XMRV). Dans ce cas, le dysfonctionnement du système immunitaire est étroitement lié à l'état physiologique ou physiopathologique du patient.

Un patient immunodéprimé ou immunodéficient ne possède pas l'intégralité des acteurs de la réponse immunitaire. Le déficit d'un ou plusieurs des lignages cellulaires du système immunitaire perturbe le système de défense du patient dans sa lutte vis-à-vis des agents pathogènes. L'immunosuppression peut altérer le lignage B et/ou le lignage T.

Un patient immunodéprimé ou immunodéficient au sens de la présente invention, séropositif pour EBV, peut présenter un profil sérologique atypique vis-à-vis des marqueurs viraux EBNA, VCA et EA. A titre d'exemple et sans que cela restreigne l'idée de la présente invention, le profil sérologique suivant est considéré comme atypique par les cliniciens : IgG anti-VCA positif/IgM anti-VCA négatif/IgA anti-VCA positif/ IgG anti-EA négatif/ IgG anti- EBNA positif. Un patient immunodéprimé ou immunodéficient, séropositif pour EBV, possède généralement des titres en anticorps anti-EBV plus faibles que ceux observés chez un patient immunocompétent, séropositif pour EBV.

Selon un mode de réalisation plus particulier, dans l'utilisation de l'invention, le patient est immunodéprimé ou immunodéficient et appartient notamment au groupe constitué par : patient en attente d'une greffe, patient receveur d'une greffe, patient séropositif pour un virus immunosuppresseur tel que VIH, ou patient présentant un déficit immunologique génétiquement transmis.

Selon un autre mode de réalisation particulier, dans la méthode de pronostic ou de diagnostic *in vitro* et/ou *ex-vivo* d'une lymphoprolifération associée à la forme réplicative d'EBV ou plus généralement d'une tumeur associée à EBV, en particulier le cancer du nasopharynx, le patient est immunodéprimé ou immunodéficient et appartient notamment au groupe constitué par : patient en attente d'une greffe, patient receveur d'une greffe, patient séropositif pour un virus immunosuppresseur tel que VIH, ou patient présentant un déficit immunologique génétiquement transmis.

Selon un mode de réalisation particulier, l'utilisation de l'invention permet le pronostic ou le diagnostic d'un premier épisode de lymphoprolifération, dans laquelle la quantité de complexe protéique varie de 1 à 100 microgrammes/ml d'échantillon biologique, ledit premier épisode de lymphoprolifération intervenant avant l'initiation de tout traitement thérapeutique d'un lymphome.

Par «premier épisode de lymphoprolifération», on désigne l'identification par le clinicien, sur la base de marqueurs biologiques et l'état général du patient, d'une lymphoprolifération. Ce premier épisode correspond au premier diagnostic de lymphoprolifération qui suit la greffe.

Par «avant l'initiation de tout traitement thérapeutique d'un lymphome», on désigne l'intervalle de temps qui s'est écoulé entre la greffe et le premier diagnostic de lymphome. Pendant cette période, le patient ne reçoit ni traitement anti-viral ni traitement ciblant la destruction d'une partie du pool des lymphocytes B infectés par EBV.

Selon un mode de réalisation particulier, l'utilisation de l'invention comprend en outre la détection des anticorps anti-ZEBRA.

Par « anticorps anti-ZEBRA », on désigne les anticorps fabriqués par le système immunitaire du patient infecté par EBV spécifiquement dirigé contre l'antigène ZEBRA. Ces anticorps sont incapables de se lier à d'autres antigènes avec la même affinité que celle qui caractérise la liaison antigène ZEBRA-anticorps anti-ZEBRA.

Le résultat de la détection des anticorps anti-ZEBRA peut être exprimé de manière relative (positif/négatif) ou sous forme de titre, c'est-à-dire correspondant à la dilution sériée limite à partir de laquelle un échantillon positif devient négatif.

Selon un mode de réalisation plus particulier, dans l'utilisation de l'invention, la charge virale d'EBV est également déterminée dans un échantillon biologique issu du susdit patient.

Par « charge virale », on désigne le nombre de copies de génome viral retrouvées dans un échantillon issu d'un patient séropositif pour EBV. Cette mesure peut être effectuée en recherchant l'un des gènes viraux présent dans le génome d'EBV à l'aide d'amorces et de sondes spécifiques. L'unité de mesure de la charge virale peut être exprimée en copies / ml d'échantillon, copies/µg d'ADN cellulaire qui est équivalent à copies/150 000 cellules (1 µg représente 150 000 cellules) ou en UI (Unité Internationale) / ml (First WHO International Standard for EBV, NIBSC 09/260).

Selon un mode de réalisation encore plus particulier, dans l'utilisation de l'invention la charge virale liée à une lymphoprolifération est environ égale ou supérieure à 10⁴ copies pour 150 000 cellules, et de préférence comprise dans une gamme variant de 10⁴ à environ 10⁹ copies pour 150 000 cellules.

Selon encore un mode de réalisation plus particulier, dans l'utilisation de l'invention la charge virale liée à une lymphoprolifération est environ égale ou supérieure à 3,2.10⁴ copies /ml de liquide biologique, ledit liquide biologique étant notamment du sang ou du sérum.

Selon un mode de réalisation particulier, l'utilisation de l'invention comprend en outre un dosage de l'IL-10 dans l'échantillon biologique.

Par «IL-10», on désigne l'interleukine-10 d'origine humaine (hIL-10) ou animale (aIL-10). La synthèse de cette cytokine peut être déclenchée par des protéines virales d'EBV. La mesure du taux d'IL-10 peut être exprimée de manière relative (négatif/positif) ou quantificative (pg/ml d'échantillon ou toute autre unité).

Selon un mode de réalisation plus particulier, dans l'utilisation de l'invention la quantité d'IL-10 liée à une lymphoprolifération est supérieure à environ 100 picogrammes/ml, et de préférence comprise dans une gamme variant de 100 à 250 picogrammes/ml.

Selon un mode de réalisation particulier, dans l'utilisation de l'invention, la mise en évidence de la protéine ZEBRA de séquence SEQ ID NO: 1 comprend en outre la détection des anticorps anti-ZEBRA, et/ou en outre le dosage de l'IL-10.

Selon un mode de réalisation encore plus particulier, l'utilisation de la présente invention, dans laquelle le patient est immunodéprimé ou immunodéficient, pour le pronostic ou le diagnostic d'un premier épisode de lymphoprolifération, dans laquelle la quantité de protéine ZEBRA SEQ ID NO:1 varie de 1 à 100 microgrammes/ml d'échantillon biologique, ledit premier épisode de lymphoprolifération intervenant avant l'initiation de tout traitement thérapeutique d'un lymphome, laquelle comprend en outre :
- la détection des anticorps anti-ZEBRA, et
- éventuellement la mesure de la charge virale d'EBV, ladite charge virale liée à un premier épisode de lymphoprolifération étant égale ou supérieure à 10⁴ copies pour 150 000 cellules, et de préférence comprise dans une gamme variant de 10⁴ à 10⁹ copies pour 150 000 cellules, et
- éventuellement le dosage de l'IL-10, la quantité d'IL-10 liée à un premier épisode de lymphoprolifération étant supérieure à environ 100 picogrammes/ml, et de préférence comprise dans une gamme variant de 100 à 250 picogrammes/ml.

La présente divulgation concerne également, selon un mode de réalisation encore plus particulier, l'utilisation de la présentedivulgation, dans laquelle le patient est immunodéprimé ou immunodéficient, pour le pronostic ou le diagnostic d'un premier épisode de lymphoprolifération, dans laquelle la quantité de complexe protéique varie de 1 à 100 microgrammes/ml d'échantillon biologique, ledit premier épisode de lymphoprolifération intervenant avant l'initiation de tout traitement thérapeutique d'un lymphome, laquelle comprend en outre :
- la détection des anticorps anti-ZEBRA, et
- éventuellement la mesure de la charge virale d'EBV, ladite charge virale liée à un premier épisode de lymphoprolifération étant environ égale ou supérieure à 10⁴ copies pour 150 000 cellules, et de préférence comprise dans une gamme variant de 10⁴ à environ 10⁹ copies pour 150 000 cellules, et
- éventuellement le dosage de l'IL-10, la quantité d'IL-10 liée à un premier épisode de lymphoprolifération étant supérieure à environ 100 picogrammes/ml, et de préférence comprise dans une gamme variant de 100 à 250 picogrammes/ml.

La présente divulgation concerne également une méthode de pronostic ou de diagnostic *in vitro* et/ou *ex-vivo,* dans laquelle le patient est immunodéprimé ou immunodéficient, pour le pronostic ou le diagnostic d'un premier épisode de lymphoprolifération, dans laquelle la quantité de complexe protéique varie de 1 à 100 microgrammes/ml d'échantillon biologique, ledit premier épisode de lymphoprolifération intervenant avant l'initiation de tout traitement thérapeutique d'un lymphome, comprend en outre :
- la détection des anticorps anti-ZEBRA, et
- éventuellement la mesure de la charge virale d'EBV, ladite charge virale liée à un premier épisode de lymphoprolifération étant environ égale ou supérieure à 10⁴ copies pour 150 000 cellules, et de préférence comprise dans une gamme variant de 10⁴ à environ 10⁹ copies pour 150 000 cellules, et
- éventuellement le dosage de l'IL-10, la quantité d'IL-10 liée à un premier épisode de lymphoprolifération étant supérieure à environ 100 picogrammes/ml, et de préférence comprise dans une gamme variant de 100 à 250 picogrammes/ml.

Selon un autre mode de réalisation plus particulier, la méthode de pronostic ou de diagnostic *in vitro* et/ou *ex-vivo* selon la présente invention, dans laquelle le patient est immunodéprimé ou immunodéficient, pour le pronostic ou le diagnostic d'un premier épisode de lymphoprolifération, dans laquelle la quantité de protéine ZEBRA SEQ ID NO:1 varie de 1 à 100 microgrammes/ml d'échantillon biologique, ledit premier épisode de lymphoprolifération intervenant avant l'initiation de tout traitement thérapeutique d'un lymphome, comprend en outre :
- la détection des anticorps anti-ZEBRA, et
- éventuellement la mesure de la charge virale d'EBV, ladite charge virale liée à un premier épisode de lymphoprolifération étant environ égale ou supérieure à 10⁴ copies pour 150 000 cellules, et de préférence comprise dans une gamme variant de 10⁴ à environ 10⁹ copies pour 150 000 cellules, et
- éventuellement le dosage de l'IL-10, la quantité d'IL-10 liée à un premier épisode de lymphoprolifération étant supérieure à environ 100 picogrammes/ml, et de préférence comprise dans une gamme variant de 100 à 250 picogrammes/ml.

Selon un mode de réalisation particulier, l'utilisation de l'invention permet le pronostic ou le diagnostic d'une réactivation de la forme réplicative d'EBV intervenant suite à un traitement du patient pour un premier épisode de lymphome associé à la réactivation d'EBV.

Par « réactivation », on désigne le passage de la forme latente d'EBV à la forme lytique. La protéine ZEBRA sert « d'interrupteur » en ce sens qu'elle va permettre la transcription des gènes précoces d'EBV. Les produits issus de ces gènes précoces vont pouvoir à leur tour induire l'expression des gènes tardifs d'EBV.

Dans le cas présent, la réactivation d'EBV intervient alors que le patient a reçu un traitement visant les lymphocytes B matures infectés par l'une des formes latentes d'EBV. Ce traitement repose notamment sur l'utilisation d'anticorps monoclonaux (MabThera/Rituximab) dirigés contre l'antigène CD20 présent à la surface des lymphocytes B matures, qu'ils soient ou non infectés par EBV. La réactivation d'EBV post-traitement du patient est susceptible d'entrainer un nouvel épisode de lymphoprolifération.

Selon un mode de réalisation plus particulier, dans l'utilisation de la présente divulgation, la quantité de complexe protéique pronostique ou diagnostique d'une réactivation de la forme réplicative d'EBV est environ égale ou supérieure à 0,15 microgrammes/ml d'échantillon biologique, et de préférence comprise dans une gamme variant de 0,15 à 20 microgrammes/ml.

Selon un mode de réalisation plus particulier, dans l'utilisation de l'invention, la quantité de complexe protéique pronostique ou diagnostique d'une réactivation de la forme réplicative d'EBV est environ égale ou supérieure à 0,15 microgrammes/ml d'échantillon biologique, et de préférence comprise dans une gamme variant de 0,15 à 20 microgrammes/ml.

Selon un mode de réalisation particulier, l'utilisation de l'invention comprend en outre la détection des anticorps anti-ZEBRA.

Selon un mode de réalisation plus particulier, dans l'utilisation de l'invention, la charge virale d'EBV est également déterminée dans un échantillon biologique issu du patient.

Selon un mode de réalisation encore plus particulier, dans l'utilisation de l'invention, la charge virale d'EBV est environ égale ou supérieure à 10³ copies pour 150 000 cellules, et de préférence comprise dans une gamme variant de 10³ à environ 10⁹ copies pour 150 000 cellules.

Selon un mode de réalisation particulier, l'utilisation de la présente divulgation dans laquelle le patient est immunodéprimé ou immunodéficient, pour le pronostic ou le diagnostic d'une réactivation de la forme réplicative d'EBV intervenant suite à un traitement du patient pour un premier épisode de lymphome associé à la réactivation d'EBV, dans laquelle la quantité de complexe protéique pronostique ou diagnostique d'une réactivation de la forme réplicative d'EBV est environ égale ou supérieure à 0,15 microgrammes/ml d'échantillon biologique, et de préférence comprise dans une gamme variant de 0,15 à 20 microgrammes/ml, laquelle comprend en outre :
- la détection des anticorps anti-ZEBRA, et
- éventuellement la mesure de la charge virale d'EBV, ladite charge virale d'EBV liée à la réactivation de la forme réplicative d'EBV étant environ égale ou supérieure à 10³ copies pour 150 000 cellules, et de préférence comprise dans une gamme variant de 10³ à environ 10⁹ copies pour 150 000 cellules.

Selon un mode de réalisation particulier, l'utilisation de la présente invention dans laquelle le patient est immunodéprimé ou immunodéficient, pour le pronostic ou le diagnostic d'une réactivation de la forme réplicative d'EBV intervenant suite à un traitement du patient pour un premier épisode de lymphome associé à la réactivation d'EBV, dans laquelle la quantité de la protéine ZEBRA SEQ ID NO:1 pronostique ou diagnostique d'une réactivation de la forme réplicative d'EBV est environ égale ou supérieure à 0,15 microgrammes/ml d'échantillon biologique, et de préférence comprise dans une gamme variant de 0,15 à 20 microgrammes/ml, laquelle comprend en outre :
- la détection des anticorps anti-ZEBRA, et
- éventuellement la mesure de la charge virale d'EBV, ladite charge virale d'EBV liée à la réactivation de la forme réplicative d'EBV étant environ égale ou supérieure à 10³ copies pour 150 000 cellules, et de préférence comprise dans une gamme variant de 10³ à environ 10⁹ copies pour 150 000 cellules.

Selon un autre mode de réalisation plus particulier, la méthode de pronostic ou de diagnostic *in vitro* et/ou *ex-vivo* selon la présente divulgation, dans laquelle le patient est immunodéprimé ou immunodéficient, pour le pronostic ou le diagnostic d'une réactivation de la forme réplicative d'EBV intervenant suite à un traitement du patient pour un premier épisode de lymphome associé à la réactivation d'EBV, dans laquelle la quantité de complexe protéique pronostique ou diagnostique d'une réactivation de la forme réplicative d'EBV est environ égale ou supérieure à 0,15 microgrammes/ml d'échantillon biologique, et de préférence comprise dans une gamme variant de 0,15 à 20 microgrammes/ml, comprend en outre :
- la détection des anticorps anti-ZEBRA, et
- éventuellement la mesure de la charge virale d'EBV, ladite charge virale d'EBV liée à la réactivation de la forme réplicative d'EBV étant environ égale ou supérieure à 10³ copies pour 150 000 cellules, et de préférence comprise dans une gamme variant de 10³ à environ 10⁹ copies pour 150 000 cellules.

Selon un autre mode de réalisation plus particulier, la méthode de pronostic ou de *diagnostic in vitro* et/ou *ex-vivo* selon la présente invention, dans laquelle le patient est immunodéprimé ou immunodéficient, pour le pronostic ou le diagnostic d'une réactivation de la forme réplicative d'EBV intervenant suite à un traitement du patient pour un premier épisode de lymphome associé à la réactivation d'EBV, dans laquelle la quantité de la protéine ZEBRA SEQ ID NO:1 pronostique ou diagnostique d'une réactivation de la forme réplicative d'EBV est environ égale ou supérieure à 0,15 microgrammes/ml d'échantillon biologique, et de préférence comprise dans une gamme variant de 0,15 à 20 microgrammes/ml, comprend en outre :
- la détection des anticorps anti-ZEBRA, et
- éventuellement la mesure de la charge virale d'EBV, ladite charge virale d'EBV liée à la réactivation de la forme réplicative d'EBV étant environ égale ou supérieure à 10³ copies pour 150 000 cellules, et de préférence comprise dans une gamme variant de 10³ à environ 10⁹ copies pour 150 000 cellules.

L'utilisation de la présente invention peut également être envisagée chez un patient immunocompétent séropositif pour EBV.

Selon un mode de réalisation particulier, dans l'utilisation de l'invention, le patient est immunocompétent et ne présente pas les signes cliniques d'une mononucléose infectieuse.

Selon un mode de réalisation plus particulier, dans l'utilisation de la présente divulgation, chez un patient immunocompétent, la quantité de complexe protéique pronostique ou diagnostique d'une réactivation de la forme réplicative d'EBV est environ égale ou supérieure à 7 microgrammes/ml d'échantillon biologique.

Selon un mode de réalisation plus particulier, dans l'utilisation de la présente invention, chez un patient immunocompétent, la quantité de protéine ZEBRA SEQ ID NO:1 pronostique ou diagnostique d'une réactivation de la forme réplicative d'EBV est environ égale ou supérieure à 7 microgrammes/ml d'échantillon biologique.

Selon un mode de réalisation particulier, l'utilisation de l'invention chez un patient immunocompétent comprend en outre la détection des anticorps anti-ZEBRA.

Selon un mode de réalisation plus particulier, dans l'utilisation de l'invention, chez un patient immunocompétent, la charge virale d'EBV est également déterminée dans un échantillon biologique issu du patient.

Selon un mode de réalisation encore plus particulier, dans l'utilisation de l'invention, chez un patient immunocompétent, la charge virale liée à une réactivation virale varie de 10² à 10³ copies pour 150 000 cellules.

Selon un mode de réalisation particulier, l'utilisation de l'invention chez un patient immunocompétent comprend en outre un dosage de l'IL-10 dans l'échantillon biologique.

Selon un mode de réalisation plus particulier, dans l'utilisation de l'invention, chez un patient immunocompétent, la quantité d'IL-10 liée à une réactivation virale est supérieure à environ 100 pg/ml, et de préférence comprise dans une gamme d'environ 100 à environ 250 pg/ml.

Selon un mode de réalisation encore plus particulier, l'utilisation de la présente divulgation dans laquelle le patient est immunocompétent et ne présente pas les signes cliniques d'une mononucléose infectieuse, dans laquelle la quantité de complexe protéique pronostique ou diagnostique d'une réactivation de la forme réplicative d'EBV est environ égale ou supérieure à 7 microgrammes/ml d'échantillon biologique, laquelle comprend en outre :
- la détection des anticorps anti-ZEBRA, et
- éventuellement la mesure de la charge virale d'EBV, ladite charge virale liée à une réactivation de la forme réplicative d'EBV variant de 10² à 10³ copies pour 150 000 cellules, et
- éventuellement le dosage de l'IL-10, la quantité d'IL-10 liée à une réactivation de la forme réplicative d'EBV étant supérieure à environ 100 pg/ml, et de préférence comprise dans une gamme d'environ 100 à environ 250 pg/ml.

Selon un mode de réalisation encore plus particulier, l'utilisation de la présente invention dans laquelle le patient est immunocompétent et ne présente pas les signes cliniques d'une mononucléose infectieuse, dans laquelle la protéine ZEBRA SEQ ID NO:1 pronostique ou diagnostique d'une réactivation de la forme réplicative d'EBV est environ égale ou supérieure à 7 microgrammes/ml d'échantillon biologique, laquelle comprend en outre :
- la détection des anticorps anti-ZEBRA, et
- éventuellement la mesure de la charge virale d'EBV, ladite charge virale liée à une réactivation de la forme réplicative d'EBV variant de 10² à 10³ copies pour 150 000 cellules, et
- éventuellement le dosage de l'IL-10, la quantité d'IL-10 liée à une réactivation de la forme réplicative d'EBV étant supérieure à environ 100 pg/ml, et de préférence comprise dans une gamme d'environ 100 à environ 250 pg/ml.

Selon un autre mode de réalisation plus particulier, la méthode de pronostic ou de diagnostic *in vitro* et/ou *ex-vivo* selon la présente divulgation, dans laquelle le patient est immunocompétent et ne présente pas les signes cliniques d'une mononucléose infectieuse, dans laquelle la quantité de complexe protéique pronostique ou diagnostique d'une réactivation de la forme réplicative d'EBV est environ égale ou supérieure à 7 microgrammes/ml d'échantillon biologique, comprend en outre :
- la détection des anticorps anti-ZEBRA, et
- éventuellement la mesure de la charge virale d'EBV, ladite charge virale liée à une réactivation de la forme réplicative d'EBV variant de 10² à 10³ copies pour 150 000 cellules, et
- éventuellement le dosage de l'IL-10, la quantité d'IL-10 liée à une réactivation de la forme réplicative d'EBV étant supérieure à environ 100 pg/ml, et de préférence comprise dans une gamme d'environ 100 à environ 250 pg/ml.

Selon un autre mode de réalisation plus particulier, la méthode de pronostic ou de diagnostic *in vitro* et/ou *ex-vivo* selon la présente invention, dans laquelle le patient est immunocompétent et ne présente pas les signes cliniques d'une mononucléose infectieuse, dans laquelle la quantité de protéine ZEBRA SEQ ID NO:1 pronostique ou diagnostique d'une réactivation de la forme réplicative d'EBV est environ égale ou supérieure à 7 microgrammes/ml d'échantillon biologique, comprend en outre :
- la détection des anticorps anti-ZEBRA, et
- éventuellement la mesure de la charge virale d'EBV, ladite charge virale liée à une réactivation de la forme réplicative d'EBV variant de 10² à 10³ copies pour 150 000 cellules, et
- éventuellement le dosage de l'IL-10, la quantité d'IL-10 liée à une réactivation de la forme réplicative d'EBV étant supérieure à environ 100 pg/ml, et de préférence comprise dans une gamme d'environ 100 à environ 250 pg/ml.

Selon encore un autre mode de réalisation, dans l'utilisation de l'invention la mise en évidence de la présence de la protéine ZEBRA SEQ ID NO :1 est répétée dans le temps, l'intervalle de temps compris entre chaque mise en évidence étant de 10 jours au plus, de préférence de 5 jours au plus, et plus préférentiellement de 3 jours.

Selon un autre mode de réalisation plus particulier, dans la méthode de pronostic ou de diagnostic *in vitro* et/ou *ex-vivo* selon la présente invention, la mise en évidence de la présence de la protéine ZEBRA SEQ ID NO:1 est répétée dans le temps, l'intervalle de temps compris entre chaque mise en évidence étant de 10 jours au plus, de préférence de 5 jours au plus, et plus préférentiellement de 3 jours.

La présente invention concerne également un kit de détection de la protéine ZEBRA SEQ ID NO:1 pour la mise en oeuvre d'un procédé tel que décrit précédemment, ledit kit de détection comprenant des anticorps spécifiquement dirigés contre la protéine ZEBRA, et notamment les anticorps AZ125 et/ou AZ130.

Selon un autre mode de réalisation, ledit kit de détection comprend en sus des anticorps spécifiquement dirigés contre la protéine ZEBRA :
- des antigènes permettant la détection des anticorps anti-ZEBRA, notamment la protéine ZEBRA sous sa forme recombinante ou des peptides dérivés de la SEQ ID NO : 1, et/ou
- des moyens de détection de la charge virale, ces moyens étant de préférence constitués par des amorces dont la séquence en acides nucléiques est choisie dans le groupe constitué par : SEQ ID NO: 2 à SEQ ID NO: 8, et/ou,
- des moyens de détection de l'IL-10, notamment des anticorps anti-IL10.

La présente invention concerne également des séquences nucléotidiques de type amorces et sondes pour la détection du virus Epstein-Barr (EBV) par amplification, et des procédés les utilisant.

L'invention concerne un oligonucléotide consistant en une séquence d'acide nucléique choisie dans le groupe comprenant les séquences SEQ ID NO: 5, SEQ ID NO: 6, et leurs séquences complémentaires.

Par « oligonucléotide », on désigne une séquence d'acide nucléique qui peut être utilisée comme amorce dans un procédé d'amplification ou comme sonde dans un procédé de détection. Dans la présente invention, les oligonucléotides consistent en une séquence d'au moins 15, préférentiellement 28 nucléotides, et préférentiellement moins de 30 nucléotides, capable de s'hybrider à une molécule d'ADN génomique ou à un ADN complémentaire. Par « hybridation », on désigne l'interaction physique existante entre deux molécules d'acide nucléique. Cette hybridation peut impliquée des homoduplex ADN/ADN ou ARN/ARN ou des hétéroduplex ADN/ARN.

Par « acide nucléique », on désigne une succession de nucléotides liés entre eux par des liaisons phosphodiester. Une molécule d'acide nucléique peut être linéaire, circulaire, simple brin, double brin, partiellement double brin. Les séquences d'acides nucléiques sont décrites dans la présente invention selon l'usage bien connu de l'homme de l'art, c'est-à-dire qu'elles sont définies par une séquence numérotée selon la direction 5' vers 3'.

Par « séquences complémentaires », on désigne deux séquences nucléiques qui possèdent des nucléotides complémentaires pouvant interagir entre eux par des liaisons hydrogènes. En face d'une adénine, il y a toujours une thymine; en face d'une cytosine, il y a toujours une guanine. A titre d'exemple, et sans que cela ne restreigne la portée de l'invention, la séquence 5' ATCG 3' et la séquence 5' CGAT 3' sont complémentaires.

L'invention concerne également l'utilisation d'oligonucléotides consistant en une séquence d'acide nucléique choisie dans le groupe comprenant les séquences SEQ ID NO: 5, SEQ ID NO: 6 et leurs séquences complémentaires, comme amorces pour réaliser une hybridation et éventuellement une amplification d'un acide nucléique provenant du génome d'EBV.

Par « amplification », on désigne l'augmentation de la concentration d'une séquence d'ADN spécifique parmi un mélange de séquences d'ADN. Les techniques d'amplification de l'ADN sont des techniques bien connues de l'homme de l'art.

L'invention concerne également l'utilisation de l'oligonucléotide consistant en la séquence SEQ ID NO: 7 ou de sa séquence complémentaire comme sonde pour réaliser une hybridation avec un acide nucléique provenant du génome d'EBV.
Par « EBV », on désigne le virus du genre *Lymphocryptovirus* de la famille des *Herpesviridae.* Le virus d'Epstein-Barr (EBV), également appelé HHV-4 (Herpès Virus Humain de type 4) possède un génome se présentant sous la forme d'un ADN linéaire double brin de 172 kilo paires de base. Il existe plusieurs sous types d'EBV appelés sous-types 1 et 2 ou A et B.

Selon un autre mode de réalisation, l'oligonucléotide consistant en la séquence SEQ ID NO: 8 ou sa séquence complémentaire peut être utilisé comme sonde pour réaliser une hybridation avec un acide nucléique provenant du génome d'EBV.

L'invention concerne également l'utilisation de l'oligonucléotide consistant en la séquence SEQ ID NO: 7 ou de sa séquence complémentaire marqué à une extrémité par un fluorophore et éventuellement à l'autre extrémité par un quencher.

Par « fluorophore », on désigne les molécules capables d'émettre de la lumière lorsqu'elles sont excitées par une source lumineuse. Les fluorophores sont des molécules bien connues par l'homme de l'art, dont les plus utilisés sont Fam, Tet, Hex, Tamra, Texas Red, Cy3, Cy5. Cette liste non exhaustive a pour but d'illustrer la notion de fluorophore mais ne doit en aucun cas restreindre la présente invention à l'utilisation de ces seuls fluorophores.

Par « quencher », on désigne une espèce chimique, capable de désactiver un état excité créé dans une entité moléculaire par transfert d'énergie, d'électron ou par un mécanisme chimique. Les quenchers sont des molécules bien connues par l'homme de l'art, dont les plus utilisés sont Dabcyl, Eclipse Dark Quencher, Black Hole Quencher. Un fluorophore peut également servir de quencher. Pour cela, le spectre d'émission du fluorophore greffé en 5' ne doit pas chevaucher le spectre d'excitation du fluorophore-quencher greffé en 3'. Cette liste non exhaustive a pour but d'illustrer la notion de quencher mais ne doit en aucun cas restreindre la présente invention à l'utilisation de ces seuls quenchers.
Dans la présente invention, les sondes sont utilisées rentrent dans la définition de la technologie Taqman. Les sondes sont marquées à leur extrémité 5' par un fluorohpore, par exemple FAM, et à leur extrémité 3' par un quencher, par exemple TAMRA, qui inhibe l'émission du fluorophore lorsqu'ils sont à proximité. Au cours de la PCR, si la sonde est hybridée sur sa cible, elle est hydrolysée par l'ADN polymérase. Le fluorophore ainsi séparé du quencher émet un signal proportionnel au nombre de sondes hydrolysées, mesurable au moment de l'élongation. Il existe d'autres technologies de PCR bien connues de l'homme de l'art, utilisant notamment le FRET (Fluorescent Resonnance Energy Transfer), les sondes de type Molecular Beacon ou de type Scorpion.

Selon un autre mode de réalisation, l'oligonucléotide consistant en la séquence SEQ ID NO: 8 ou sa séquence complémentaire marqué à une extrémité par un fluorophore et éventuellement à l'autre extrémité par un quencher peut également être utilisé.

Un autre but de l'invention est de proposer un set d'oligonucléotides consistant en les oligonucléotides de séquences SEQ ID NO: 5, SEQ ID NO: 6 et SEQ ID NO: 7.

Selon un autre mode de réalisation, le susdit set d'oligonucléotides peut consister en les oligonucléotides de séquences SEQ ID NO: 5, SEQ ID NO: 6 et SEQ ID NO: 8.

L'invention concerne également un procédé de détection d'EBV par amplification *in vitro* à partir d'un échantillon biologique, ledit procédé comportant les étapes de :
- mise en contact du set d'oligonucléotides tel que défini ci-dessus avec un échantillon biologique, ou un acide nucléique issu du susdit échantillon biologique, dans des conditions permettant aux oligonucléotides de s'hybrider à un acide nucléique d'EBV présent dans le susdit échantillon,
- amplification de l'acide nucléique d'EBV en utilisant les oligonucléotides comme amorces,
- détection du produit d'amplification caractérisant la présence d'EBV dans l'échantillon.

Selon un autre mode de réalisation, dans le procédé de détection d'EBV, l'acide nucléique d'EBV est amplifié par PCR. La PCR peut être qualitative, quantitative ou semi-quantitative. Suivant qu'une sonde de détection est utilisée ou pas, on parle de PCR en temps réel ou de PCR classique.

Selon un autre mode de réalisation plus particulier, dans le procédé de détection d'EBV, la détection du produit d'amplification est réalisée en utilisant l'oligonucléotide de séquence SEQ ID NO: 7 ou de sa séquence complémentaire marqué à une extrémité par un fluorophore et à l'autre extrémité par un quencher comme sonde.

Selon un autre mode de réalisation plus particulier, dans le procédé de détection d'EBV, la détection du produit d'amplification est réalisée en utilisant l'oligonucléotide de séquence SEQ ID NO: 8 ou de sa séquence complémentaire, marqué à une extrémité par un fluorophore et à l'autre extrémité par un quencher, comme sonde.

L'invention concerne également un kit d'amplification d'EBV à partir d'un échantillon biologique, ledit kit comprenant l'un des susdits sets d'oligonucléotides ou leurs séquences complémentaires et des moyens permettant l'amplification d'un acide nucléique d'EBV.

Selon un mode de réalisation particulier, ledit kit d'amplification comprend :
- au moins un set d'oligonucléotides consistant en les oligonucléotides de séquences SEQ ID NO: 5, SEQ ID NO: 6 et SEQ ID NO: 7,
- un moyen pour amplifier un acide nucléique d'EBV,
- éventuellement un contrôle interne.

Selon un mode de réalisation particulier, ledit kit d'amplification comprend :
- au moins un set d'oligonucléotides consistant en les oligonucléotides de séquences SEQ ID NO: 5, SEQ ID NO: 6 et SEQ ID NO: 8,
- un moyen pour amplifier un acide nucléique d'EBV,
- éventuellement un contrôle interne.

Par « moyen pour amplifier un acide nucléique », on désigne les dNTPs, la Taq Polymérase, les sels et tampons qui permettent la réalisation d'une PCR.

Par « contrôle interne », on désigne une séquence nucléique (ADN exogène) sans rapport avec le génome d'EBV, des amorces et une sonde permettant l'amplification et la détection de cet ADN exogène. Ce contrôle interne est placé dans le mix servant à la PCR pour la détection d'EBV et atteste du bon fonctionnement de l'amplification.

### Légende des figures

**Figure 1** **:** Représentation schématique des différents domaines de la protéine ZEBRA. La séquence protéique de la protéine ZEBRA compte 245 acides aminés. Les différents domaines de la protéine sont représentés par les blocs :
- A : domaine d'activation, qui s'étend des acides aminés 1 à 140,
- B : domaine de liaison à l'ADN, qui s'étend des acides aminés 176 à 194,
- C : domaine de dimérisation, qui s'étend des acides aminés 195 à 221.

Les flèches pointent les épitopes antigéniques de la protéine ZEBRA spécifiquement reconnus par les anticorps AZ125 et AZ130.

La présente invention est illustrée par les exemples suivants.

### Exemple 1 : Dosage de l'antigène ZEBRA circulant et des anticorps anti-ZEBRA chez des patients non immunodéprimés

### 1 - Patients

Une cohorte de 14 patients non immunodéprimés (Consultations MST), séropositifs pour EBV mais ne présentant pas de PTLD a été testée et la présence de l'antigène ZEBRA ou d'anticorps anti-ZEBRA a été évaluée.

### 2 - Méthode de dosage de l'antigène ZEBRA

La technique de détection de l'antigène ZEBRA soluble est basée sur la méthode ELISA, en cinq étapes: (i) coating d'une plaque 96 puits par l'anticorps AZ 125 (purifié, dilué dans du PBS à une concentration finale de 10 µg/ml final). La plaque est alors placée une nuit à 4-8°C. (ii) La plaque est lavée trois fois à l'aide d'une solution de PBS contenant 0,5% de Tween 20. On procède ensuite à la saturation des sites non spécifiques des puits par addition de tampon de saturation (PBS-Tween 20 0,05%- SVF 10%-BSA 2%) pendant 60 minutes à température ambiante sous agitation. La plaque est à nouveau rincée trois fois. (iii) 100µl de sérum préalablement dilué au 1/10 dans du tampon de dilution (PBS-Tween 20 0,05%-SVF 10%) sont ajoutés en double. En parallèle, une gamme étalon de la protéine ZEBRA est réalisée. (iv) Après lavage, 50 µl de l'anticorps AZ 130 biotinylé sont ajoutés simultanément à une concentration de 5µg/ml. La plaque est incubée sous agitation à température ambiante pendant 2 heures. Après trois lavages, la détection est réalisée par l'avidine-phosphatase alcaline (Invitrogen) diluée au 1/500 dans du PBS-Tween 20 0,05%- BSA 1%. L'incubation se fait pendant 60 minutes à température ambiante sous agitation. Les puits sont ensuite lavés quatre fois avec le même tampon PBS Tween 20 0,1%. (v) La révélation se fait à l'aide d'un substrat chromogène, le p-nitrophénylphosphate, (Sigma). Ainsi le pNPP se présente sous forme de tablettes qu'il faut diluer dans du tampon diéthanolamine (pH= 9.5) pour obtenir une concentration de 1 mg /ml. 100 µl/puits de cette solution sont ajoutés. La réaction doit se développer pendant 45 mn à l'obscurité. La lecture de la DO se fait à 405 nm (absorbance de référence à 630 nm).

### 3 - Méthode de dosage des anticorps anti-ZEBRA

Le dosage des anticorps anti-ZEBRA est réalisé d'après un test ELISA décrit par Marechal et al., 1993 Res. Virol. 144 : 397-404. La protéine ZEBRA recombinante provient d'une souche d'*Escherichia coli* transformée par le plasmide pET3c contenant la partie codante du gène *BZLF1* sous le contrôle d'une unité de transcription de type T7 polymérase. La protéine recombinante ZEBRA est coatée au fond des puits d'une microplaque et sert d'hameçon aux anticorps anti-ZEBRA. Le titre des anticorps anti-ZEBRA est défini en utilisant la méthode de dilution en point final décrite par Vaur et al. 1986, J. Clin. Microbiol., 24 : 596-599.

### 4 - Résultats

La mesure de la concentration de l'antigène ZEBRA ainsi que la titration des anticorps anti-ZEBRA ont été effectuées sur des échantillons de sérums provenant des 14 patients de la cohorte ont été testés selon les techniques précisées ci-dessus. Les résultats figurent dans le tableau 1 ci-dessous.

**Tableau 1 : détection de l'antigène ZEBRA et des anticorps anti-ZEBRA dans le sérum de patients immunocompétents.**

| **Patients** | **ZEBRA antigène (µg/ml)** | **Anti-ZEBRA IgG (dilution)** |
|---|---|---|
| #80 | <0,15 | 512 |
| #81 | <0,15 | 256 |
| #82 | <0,15 | 512 |
| #83 | <0,15 | 128 |
| #84 | <0,15 | 128 |
| #87 | <0,15 | 64 |
| #88 | <0,15 | 1024 |
| #89 | <0,15 | 16384 |
| #91 | <0,15 | 1024 |
| #92 | <0,15 | 64 |
| #93 | <0,15 | 128 |
| #94 | <0,15 | <64 |
| #95 | <0,15 | 256 |
| #96 | <0,15 | 256 |

L'antigène ZEBRA est trouvé à une concentration inférieure à 0,15 µg/ml dans le sérum des 14 patients testés. La présence d'anticorps anti-ZEBRA est détectée à des titres très variables d'un patient à l'autre. Chez les patients #88, #89 et #91, EBV connait une phase de réplication plus active que chez les patients #87 et #92. Il est généralement admis qu'à partir d'un titre en anticorps anti-ZEBRA de 128, EBV est en phase de réplication.

Ces résultats montrent que les 14 patients immunocompétents ne présentant pas de PTLD de la cohorte sont bien tous séropositifs pour le virus EBV mais ne présentent pas de réplication forte du virus mesurable par la quantité d'antigène ZEBRA circulant.

### Exemple 2 : Dosage de l'antigène ZEBRA circulant, des anticorps anti-ZEBRA et de la charge virale EBV chez des patients immunodéprimés ne présentant pas de PTLD

### 1 - Patients

Trois patients séropositifs pour EBV et ayant reçu une greffe de moëlle mais ne présentant aucun signe clinique de lymphoprolifération a été testée et la présence de l'antigène ZEBRA ou d'anticorps anti-ZEBRA ainsi que la mesure de la charge virale EBV ont été évaluées.

### 2 - Méthode de dosage de l'antigène ZEBRA et des anticorps anti-ZEBRA

Les méthodes ont été décrites avec précision dans l'exemple 1 ci-dessus.

### 3 - Mesure de la charge virale EBV

### 3.1 - Extraction des ADN

L'extraction des ADN totaux est effectuée à l'aide d'un kit MagNA Pure Compact Nucleic Acid Isolation (Roche-Applied). Brièvement, les cellules mononuclées (PBMC) sont isolées par centrifugation sur Ficoll-Hypaque de densité 1,077 à partir d'un échantillon de sang total prélevé sur EDTA. Les PBMC sont lysés avec une solution contenant des sels chaotropiques et de la protéinase K et les acides nucléiques sont capturés à la surface de billes magnétiques (Glass Magnetic Particles). Des lavages successifs permettent d'éluer les substances non liées et l'ADN est élué avec un tampon salin à faible concentration.

### 3.2 - Gammes standard

**Trois** gammes standard sont établies à partir de dilutions sériées de constructions plasmidiques comprenant des produits de PCR se rapportant aux gènes BALF-5 ou ZEBRA. Un ADN double brin de 90 pb pour le gène BALF-5 et de 100 pb pour le gène ZEBRA ont été clonés chacun dans un vecteur pCR2.1.

Les gammes standard BALF-5 et ZEBRA s'étendent de 10³ à 10⁸ copies/ml.

Les pentes des gammes standard sont respectivement :
- de -3,47 avec un intercept à 47,42 et une valeur de R² de 0,997869 pour BALF-5,
- de -3,41 avec un intercept à 46,62 et une valeur de R² de 0,998847 pour ZEBRA (sonde de SEQ ID NO : 7), et
- de -3,49 avec un intercept à 47,42 et une valeur de R² de 0,997320 pour ZEBRA (sonde de SEQ ID NO : 8).
Ces résultats démontrent la parfaite linéarité de ces deux gammes standard. L'efficacité de PCR est respectivement de 94% pour BALF-5 et de 97% (sonde de SEQ ID NO : 7) ou 93% (sonde de SEQ ID NO : 8) pour ZEBRA.

### 3.3 - PCR

Le couple d'amorces et la sonde spécifiques du gène BALF-5 codant pour l'ADN polymérase d'EBV ont déjà été décrits dans la littérature (Kimura et al., J. Clin. Microbiol. 1999 January; 37(1): 132-136).

Un couple d'amorces et une sonde ont été spécifiquement designés par les inventeurs pour ZEBRA. Les séquences sont dans le tableau 2 ci-dessous :

**Tableau 2 : séquences des amorces et des sondes utilisées pour la détection de la charge virale EBV.**

| | | |
|---|---|---|
| Balf-5 (F) | SEQ ID NO : 2 | 5'- CGGAAGCCCTCTGGACTTC - 3' |
| Balf-5 (R) | SEQ ID NO : 3 | 5'- CCCTGTTTATCCGATGGAATG - 3' |
| Balf-5 (S) | SEQ ID NO : 4 | 5'-FAM TGTACACGCACGAGAAATGCGCCTAMRA- 3' |
| ZEBRA (F) | SEQ ID NO : 5 | 5'- CCAGGCTTGGGCACATCT - 3' |
| ZEBRA (R) | SEQ ID NO : 6 | 5'- CCCATCTAAACGCCTGATTTTT - 3' |
| ZEBRA (S) | SEQ ID NO : 7 | 5'-FAM CATTTTCAGATGATTTGGCAGCAGCCACTAMRA- 3' |
| ZEBRA (S) | SEQ ID NO : 8 | 5'-FAM CTTCAACAGGAGGCGC TAMRA- 3' |

Le mix réactionnel BALF-5 comporte 5 pmole de sonde de séquence SEQ ID NO : 4, 7,5 pmole d'amorce sens de séquence SEQ ID NO : 2, 7,5 pmole d'amorce anti-sens de séquence SEQ ID NO : 3, 1X de Taq Man Universal PCR Master Mix (Applied Biosystems) et 10% de Pré-Mix Cl Simplexa dilué au 1/100.

Le mix réactionnel ZEBRA comporte 5 pmole de sonde de séquence SEQ ID NO : 7, 7,5 pmole d'amorce sens de séquence SEQ ID NO : 5, 7,5 pmole d'amorce anti-sens de séquence SEQ ID NO : 6, 1X de Taq Man Universal PCR Master Mix (Applied Biosystems) et 10% de Pré-Mix Cl Simplexa dilué au 1/100.

Le thermoprofil d'amplification est de :
- 1 cycle de 2 minutes à 50°C,
- 1 cycle de 10 minutes à 95°C,
- 45 cycles de [15 secondes à 95°C puis 1 minute à 60°C].

Les amplifications sont réalisées sur un appareil ABI Prism 7500 (Applied Biosystems).

La taille des amplicons obtenus est respectivement de 90 pb pour Balf-5 et de 100 pb pour ZEBRA.

### 4 - Résultats

La mesure de la concentration de l'antigène ZEBRA, la titration des anticorps anti-ZEBRA et la mesure de la charge virale EBV ont été effectuées sur des échantillons de sérums provenant des 3 patients ont été testés selon les techniques précisées ci-dessus. Les résultats figurent dans le tableau 3 ci-dessous.

**Tableau 3 : détection de l'antigène ZEBRA, des anticorps anti-ZEBRA et de la charge virale EBV dans le sérum de patients immunodéprimés ne développant pas de PTLD.**

| **Patients** | **Date** | **Charge virale EBV (copies/ml)** | **ZEBRA antigène (µg/ml)** | **Anti-ZEBRA IgG (dilution)** |
|---|---|---|---|---|
| #1=AK | 12/01/2010 | *greffe* | | |
| | 15/02/2010 | 0 | <0,15 | 1024 |
| | 23/04/2011 | 925 | <0,15 | 1024 |
| #2=BER | 23/09/2009 | *greffe* | | |
| | 12/10/2009 | 0 | <0,15 | 64 |
| | 09/12/2009 | 0 | <0,15 | 512 |
| #3=AUG | 15/09/2009 | *greffe* | | |
| | 05/10/2009 | 0 | <0,15 | 256 |
| | 28/12/2009 | 7210 | <0,15 | 256 |

Pour les patients #1 et #3, la charge virale EBV, indétectable le mois suivant la greffe, atteint de l'ordre de 10³ à 10⁴ copies/ml cent jours après que la greffe a eu lieu. Parallèlement, la quantité d'anticorps anti-ZEBRA ne varie pas pendant cet intervalle de temps. Enfin, l'antigène ZEBRA est à une concentration inférieure à 0,15 µg/ml, ce qui signifie qu'EBV n'est pas en phase de réplication forte. Malgré l'augmentation de la charge virale, il n'apparait donc pas nécessaire de traiter ces patients pour prévenir un lymphome.

Pour le patient #2, la charge virale demeure nulle et l'antigène ZEBRA est à une concentration inférieure à 0,15 µg/ml non seulement dans le mois qui suit la greffe mais également 77 jours après que la greffe a eu lieu. En revanche, le patient possède un titre en anticorps anti-ZEBRA compris entre 64 et 512 qui indique que non seulement le patient est bien séropositif pour EBV mais que très probablement le virus est en début de réplication forte dans les cellules hôtes.

### Exemple 3 : Dosage de l'antigène ZEBRA circulant, des anticorps anti-ZEBRA, de la charge virale EBV et de l'IL-10 chez des patients immunodéprimés présentant un PTLD

### 1- Patients

Quatre patients séropositifs pour EBV, ayant reçu une greffe de moelle et pour lesquels un diagnostic de lymphoprolifération a été posé ont été étudiés. La présence de l'antigène ZEBRA ou d'anticorps anti-ZEBRA ainsi que la mesure de la charge virale EBV et la concentration d'IL-10 ont été évaluées. Une étude cinétique de ces paramètres biologiques a été réalisée.

### 2 - Méthode de dosage de l'antigène ZEBRA et des anticorps anti-ZEBRA

Les méthodes sont décrites avec précision dans l'exemple 1 ci-dessus.

### 3 - Mesure de la charge virale EBV

Les méthodes sont décrites avec précision dans l'exemple 2 ci-dessus.

### 4 - Dosage de l'IL-10

Le dosage de l'IL-10 dans les échantillons de patients est réalisé à l'aide du kit commercial Human IL-10 CytoSet^{™} (Invitrogen, Camarillo, USA) selon une méthode ELISA en cinq étapes : (i) coating d'une microplaque 96 puits par l'anticorps anti-Human IL-10 à raison de 0,1 µg/puits. La microplaque est incubée à 4°C pendant 12 à 18h. (ii) lavage de la microplaque puis incubation de 300 µl/puits de solution Assay Buffer à température ambiante pendant une heure. (iii) ajout de 100 µl de standard ou d'échantillons et de 50 µl d'anticorps de détection couplé à la biotine / puits et incubation sous agitation à température ambiante pendant 2 heures. (iv) la microplaque est rincée 5 fois et 100 µl de streptavidine-HRP /puits sont incubés sous agitation à température ambiante pendant 30 minutes. (v) la microplaque est rincée 5 fois et 100 ul de substrat chromogénique TMB/puits sont incubés sous agitation à température ambiante pendant 30 minutes. A l'issue de cette période d'incubation 100 µl de solution d'arrêt sont ajoutés dans chacun des puits. La lecture de la DO se fait à 450 nm dans les 30 minutes qui suivent l'ajout de la solution d'arrêt.

### 5 - Résultats

La mesure de la concentration de l'antigène ZEBRA, la titration des anticorps anti-ZEBRA, la mesure de la charge virale EBV et le dosage de l'IL-10 ont été effectués sur des échantillons de sérums provenant des 4 patients selon les techniques précisées ci-dessus. Les résultats figurent dans le tableau 4 ci-dessous.

**Tableau 4 : détection de l'antigène ZEBRA, des anticorps anti-ZEBRA, de la charge virale EBV et de la concentration en IL-10 dans le sérum de patients immunodéprimés développant un PTLD.**

| | **Charge virale EBV (copies/150 000 cellules)** | **ZEBRA antigène (µg/ml)** | **Anti-ZEBRA IgG (dilution)** | **IL-10 (pg/ml)** |
|---|---|---|---|---|
| **Patient # 1** | | | | |
| J0 | *Greffe* | | | |
| J21 | 0 | <0,15 | <64 | 0 |
| J36 | 2,5x10⁴ | <0,15 | <64 | 0 |
| J56 | 2,0x10⁶ | 8,49 | 256 | 0 |
| J61 | 1,0x10⁴ | 1,62 | 0 | 0 |
| J68 | 1,2x10⁴ | <0,15 | 64 | 0 |
| J71 | 1,7x10⁴ | <0,15 | <64 | 100 |
| J75 | 3,9x10⁴ | <0,15 | 64 | 0 |
| J82 | 1,6x10⁴ | 7,61 | 64 | 0 |
| J89 | 1,5x10³ | <0,15 | 64 | 0 |
| J93 | 6,9x10³ | <0,15 | 128 | 0 |
| J96 | 1,2x10³ | <0,15 | 64 | 0 |
| J103 | 1,9x10³ | <0,15 | 256 | 0 |
| J110 | 1,7x10³ | 1,54 | ND | 0 |
| J124 | 0 | 8,32 | 64 | ND |

| **Patient # 2** | | | | |
|---|---|---|---|---|
| J0 | *Greffe* | | | |
| J88 | 0 | 96,5 | 512 | 220 |
| J102 | 5,7x10³ | 36,17 | <64 | 150 |
| J141 | 1,7x10³ | <0,15 | <64 | 0 |
| J144 | 2,9x10³ | <0,15 | <64 | 120 |
| J151 | 6,3x10³ | <0,15 | 64 | 0 |
| J159 | 6,0x10⁵ | <0,15 | 64 | 150 |

| **Patient # 3** | | | | |
|---|---|---|---|---|
| J0 | *Greffe* | | | |
| J11 | 5,3x10² | <0,15 | 128 | 70 |
| J14 | 1,9x10³ | <0,15 | 512 | 70 |
| J18 | 1,7x10⁴ | 4,59 | <64 | 0 |
| J32 | ND | 1,71 | ND | ND |
| J39 | 7,5x10⁴ | <0,15 | 256 | 0 |

| **Patient # 4** | | | | |
|---|---|---|---|---|
| J0 | *Greffe* | | | |
| J14 | 0 | <0,15 | 128 | ND |
| J21 | ND | <0,15 | ND | ND |
| J29 | ND | <0,15 | ND | ND |
| J32 | ND | <0,15 | ND | ND |
| J35 | 1,3x10⁴ | 7,48 | 1024 | ND |
| J38 | ND | <0,15 | ND | ND |
| J42 | 1,2x10⁴ | <0,15 | 512 | ND |
| J63 | ND | <0,15 | ND | ND |
| J72 | ND | <0,15 | ND | ND |
| J80 | 2,1x10⁴ | <0,15 | 512 | ND |
| J95 | ND | <0,15 | 256 | ND |
| J102 | 1,2x10⁴ | <0,15 | 256 | ND |
| J112 | ND | 23,24 | ND | ND |
| J121 | ND | 23,65 | ND | ND |
| J135 | ND | 1,62 | ND | ND |
| J143 | ND | 3,14 | ND | ND |

### Patient #1 :

Trois semaines après que la greffe a eu lieu, tous les marqueurs biologiques testés sont au plus bas.

A J36, seule la charge virale EBV varie pour atteindre 2,5.10⁴ copies/150 000 cellules. A J56, la charge virale EBV atteint 2.10⁶ copies/150 000 cellules, l'antigène ZEBRA est détecté à 8,49 µg/ml et le titre en anticorps anti-ZEBRA a augmenté à 256. Ces marqueurs sont le signe clinique d'une réplication forte d'EBV argumentée par le pic d'antigène ZEBRA, l'élévation du taux d'anticorps anti-ZEBRA en association avec une augmentation du pool de lymphocytes B infectés par EBV (charge virale EBV). Un diagnostic de PTLD est posé à J56 et le patient est traité par Mabthéra™ à raison d'une cure par semaine pendant quatre semaines.

De J61 à J75, la charge virale EBV, le taux d'antigène ZEBRA et la quantité d'anticorps anti-ZEBRA diminuent et se stabilisent à des valeurs proches de celles trouvées à J36. Ceci signifie que la réplication virale a chuté. On observe toutefois à J71 un pic de synthèse d'IL-10.

A J82, l'antigène ZEBRA est détecté à une valeur de 7,61 µg/ml alors que ni la charge virale EBV ni le titre en anticorps anti-ZEBRA n'ont été modifiés par rapport à J68. Cette augmentation de la concentration en antigène ZEBRA indique qu'il existe une reprise de la réplication forte d'EBV.

Alors que la charge virale EBV évolue peu entre J89 et J110, le titre en anticorps anti-ZEBRA augmente par pics à J93 et à J103.

A J110, la concentration de l'antigène ZEBRA augmente à 1,54 µg/ml, signe d'une nouvelle phase de réplication forte d'EBV. Au même moment, un nouvel épisode de PTLD est diagnostiqué. Le patient est traité par chimio et radiothérapie avant de recevoir une seconde greffe à J124. Ces traitements ont pour conséquence une chute du titre des anticorps anti-ZEBRA.

### Patient #2 :

A J88, alors que la charge virale EBV est nulle, la concentration en antigène ZEBRA atteint 96,5 µg/ml, le titre en anticorps anti-ZEBRA est de 512 et l'IL-10 est à 220 pg/ml. Ces marqueurs sont le signe d'une réplication forte d'EBV.

A J102, la charge virale EBV atteint 5,7.10³ copies/150 000 cellules, signe qu'un plus grand nombre de lymphocytes B sont infectés par EBV.

A J141 et J144, bien que la charge virale EBV reste stable, on observe une diminution non seulement du titre en anticorps anti-ZEBRA mais également une diminution de la concentration en antigène ZEBRA. Ceci est le reflet d'une chute de l'activité réplicative d'EBV. Parallèlement à J144, un pic de sécrétion d'IL-10 est observé.

A J159, la charge virale EBV augmente de deux logs et un nouveau pic d'IL-10 est détecté. Un diagnostic de PTLD est posé et le patient est traité par Mabthéra™.

### Le patient #3

Deux semaines après que la greffe a eu lieu (J14), le titre en anticorps anti-ZEBRA augmente pour atteindre 512. Ceci est le reflet d'un début de réplication forte d'EBV. Cette hypothèse est confirmée à J18 par l'augmentation du taux d'antigène ZEBRA qui passe de <0,15 µg/ml à 4,59 µg/ml et par l'augmentation de la charge virale EBV d'un log en quatre jours.

A J39, un diagnostic de PTLD est posé.

### Le patient #4

A J35, la charge virale EBV atteint 1,3.10⁴ copies/150 000 cellules, l'antigène ZEBRA est détecté à 7,48 µg/ml et le titre en anticorps anti-ZEBRA a augmenté à 1024. Ces marqueurs sont le signe clinique d'une réplication forte d'EBV argumentée par le pic d'antigène ZEBRA, l'élévation du taux d'anticorps anti-ZEBRA en association avec une augmentation du pool de lymphocytes B infectés par EBV (charge virale EBV). Un diagnostic de PTLD est posé à J35 et le patient est traité par Mabthéra™ à raison d'une cure par semaine pendant quatre semaines.

A partir de J112, le taux d'antigène ZEBRA varie de 23 à 1,62 µg/ml, suggérant une reprise de la réplication forte d'EBV.

## Revendications

1. Méthode de pronostic ou de diagnostic *in vitro* et/ou *ex-vivo* d'une lymphoprolifération associée à la forme réplicative d'EBV ou plus généralement d'une tumeur associée à EBV, en particulier le cancer du nasopharynx, ladite méthode nécessitant la mise en évidence de la présence de la protéine ZEBRA de séquence SEQ ID NO: 1, dans un échantillon biologique choisi parmi le groupe constitué par le sang et le sérum, d'un patient séropositif pour EBV,
ladite mise en évidence de la protéine ZEBRA de séquence SEQ ID NO: 1 comprenant l'étape suivante :
- détermination de la présence d'un complexe immun dans un échantillon biologique préalablement prélevé d'un patient séropositif pour EBV, ledit complexe immun étant constitué de la susdite protéine liée aux ligands suivants : anticorps monoclonal dirigé contre le peptide 1-140 de la protéine ZEBRA de SEQ ID NO : 1 AZ125 et anticorps monoclonal AZ130 pris en combinaison, l'anticorps AZ130 étant l'anticorps sécrété par l'hybridome déposé à la CNCM (Collection Nationale de Culture de Microorganismes, Institut Pasteur, 25 rue du Docteur Roux, 75724 Paris Cedex 15, France) sous le traité de Budapest le 17 novembre 2011, sous la référence CNCM I-4554,
ladite quantité de protéine ZEBRA de séquence SEQ ID NO: 1 pronostique ou diagnostique d'une lymphoprolifération étant notamment égale ou supérieure à 0,15 microgrammes/ml d'échantillon biologique.

2. Méthode selon la revendication 1, dans laquelle la liaison de la protéine ZEBRA de séquence SEQ ID NO : 1 à l'un des ligands est détectée à l'aide d'un test de type immunoessai, notamment de type ELISA sandwich dans lequel l'anticorps AZ125 est adsorbé sur une plaque appropriée et l'anticorps AZ130 est utilisé pour la détection de la fixation de la protéine ZEBRA de séquence SEQ ID NO : 1 à l'anticorps AZ125 adsorbé.

3. Méthode selon la revendication 1 ou 2, dans laquelle la mise en évidence de la protéine ZEBRA de séquence SEQ ID NO: 1 comprend en outre la détection des anticorps anti-ZEBRA, et/ou en outre le dosage de l'IL-10.

4. Méthode selon l'une des revendications 1 à 3, dans laquelle la mise en évidence de la protéine ZEBRA de séquence SEQ ID NO: 1 comprend en outre la mesure de la charge virale d'EBV, notamment par une technique de type PCR, notamment en utilisant des amorces et des sondes dont les séquences en acides nucléiques sont choisies dans le groupe constitué par : SEQ ID NO: 2, SEQ ID NO: 3, SEQ ID NO: 4, SEQ ID NO: 5, SEQ ID NO: 6, SEQ ID NO: 7, SEQ ID NO: 8.

5. Méthode selon les revendications 1 à 4 dans laquelle le statut immunologique du patient appartient au groupe : patient immunocompétent, patient immunodéprimé ou patient immunodéficient, le susdit patient immunodéprimé ou le susdit patient immunodéficient appartenant notamment au groupe constitué par : patient en attente d'une greffe, patient receveur d'une greffe, patient séropositif pour un virus immunosuppresseur tel que VIH, ou patient présentant un déficit immunologique génétiquement transmis.

6. Méthode selon la revendication 5, dans laquelle le patient est immunodéprimé ou immunodéficient, pour le pronostic ou le diagnostic d'un premier épisode de lymphoprolifération, dans laquelle la quantité de protéine ZEBRA de séquence SEQ ID NO: 1 varie de 1 à 100 microgrammes/ml d'échantillon biologique, ledit premier épisode de lymphoprolifération intervenant avant l'initiation de tout traitement thérapeutique d'un lymphome, laquelle comprend en outre :
- la détection des anticorps anti-ZEBRA, et
- éventuellement la mesure de la charge virale d'EBV, ladite charge virale liée à un premier épisode de lymphoprolifération étant égale ou supérieure à 10⁴ copies pour 150 000 cellules, et de préférence comprise dans une gamme variant de 10⁴ à 10⁹ copies pour 150 000 cellules, et
- éventuellement le dosage de l'IL-10, la quantité d'IL-10 liée à un premier épisode de lymphoprolifération étant supérieure à 100 picogrammes/ml, et de préférence comprise dans une gamme variant de 100 à 250 picogrammes/ml.

7. Méthode selon la revendication 5, dans laquelle le patient est immunodéprimé ou immunodéficient, pour le pronostic ou le diagnostic d'une réactivation de la forme réplicative d'EBV intervenant suite à un traitement du patient pour un premier épisode de lymphome associé à la réactivation d'EBV, dans laquelle la quantité de protéine ZEBRA de séquence SEQ ID NO: 1 pronostique ou diagnostique d'une réactivation de la forme réplicative d'EBV est égale ou supérieure à 0,15 microgrammes/ml d'échantillon biologique, et de préférence comprise dans une gamme variant de 0,15 à 20 microgrammes/ml, laquelle comprend en outre :
- la détection des anticorps anti-ZEBRA, et
- éventuellement la mesure de la charge virale d'EBV, ladite charge virale d'EBV liée à la réactivation de la forme réplicative d'EBV étant égale ou supérieure à 10³ copies pour 150 000 cellules, et de préférence comprise dans une gamme variant de 10³ à 10⁹ copies pour 150 000 cellules.

8. Méthode selon la revendication 5, dans laquelle le patient est immunocompétent et ne présente pas les signes cliniques d'une mononucléose infectieuse, dans laquelle la quantité de protéine ZEBRA de séquence SEQ ID NO: 1 pronostique ou diagnostique d'une réactivation de la forme réplicative d'EBV est égale ou supérieure à 7 microgrammes/ml d'échantillon biologique, laquelle comprend en outre :
- la détection des anticorps anti-ZEBRA, et
- éventuellement la mesure de la charge virale d'EBV, ladite charge virale liée à une réactivation de la forme réplicative d'EBV variant de 10² à 10³ copies pour 150 000 cellules, et
- éventuellement le dosage de l'IL-10, la quantité d'IL-10 liée à une réactivation de la forme réplicative d'EBV étant supérieure à 100 pg/ml, et de préférence comprise dans une gamme de 100 à 250 pg/ml.

9. Méthode selon l'une des revendications 1 à 8 dans laquelle la mise en évidence de la présence de la protéine ZEBRA de séquence SEQ ID NO: 1 est répétée dans le temps, l'intervalle de temps compris entre chaque mise en évidence étant de 10 jours au plus, de préférence de 5 jours au plus, et plus préférentiellement de 3 jours.

10. Kit de détection de la protéine ZEBRA de séquence SEQ ID NO: 1 pour la mise en oeuvre de la mise en évidence de la présence de la protéine ZEBRA de séquence SEQ ID NO: 1 définie selon l'une des revendications 1 à 4, ledit kit de détection comprenant les anticorps AZ125 et AZ130.

11. Kit de détection selon la revendication 10, ledit kit de détection comprenant en sus des anticorps spécifiquement dirigés contre la protéine ZEBRA :
- des antigènes permettant la détection des anticorps anti-ZEBRA, notamment la protéine ZEBRA sous sa forme recombinante ou des peptides dérivés de la SEQ ID NO: 1, et/ou
- des moyens de détection de la charge virale, ces moyens étant de préférence constitués par des amorces dont la séquence en acides nucléiques est choisie dans le groupe constitué par : SEQ ID NO: 2 à SEQ ID NO: 8, et/ou,
- des moyens de détection de l'IL-10, notamment des anticorps anti-IL10.

12. Anticorps AZ130 qui lie spécifiquement le fragment C terminal de la protéine ZEBRA de séquence SEQ ID NO: 1, l'anticorps AZ130 étant l'anticorps sécrété par l'hybridome déposé à la CNCM (Collection Nationale de Culture de Microorganismes, Institut Pasteur, 25 rue du Docteur Roux, 75724 Paris Cedex 15, France) sous le traité de Budapest le 17 novembre 2011, sous la référence CNCM I-4554.

13. Hybridome déposé à la CNCM (Collection Nationale de Culture de Microorganismes, Institut Pasteur, 25 rue du Docteur Roux, 75724 Paris Cedex 15, France) sous le traité de Budapest le 17 novembre 2011, sous la référence CNCM I-4554, capable de sécréter l'anticorps AZ130 tel que défini à la revendication 12.

## Patentansprüche

1. Verfahren zur in vitro- und/oder ex-vivo-Prognose oder zur Diagnose einer Lymphoproliferation, die mit der replikativen Form von EBV assoziiert ist, oder allgemeiner eines Tumors, der mit EBV assoziiert ist, insbesondere Nasen-Rachen-Krebs, wobei das Verfahren den Nachweis des Vorhandenseins des ZEBRA-Proteins mit der Sequenz SEQ ID NR.: 1 in einer biologischen Probe, ausgewählt aus der Gruppe bestehend aus But und Serum, eines für EBV seropositiven Patienten erfordert,
wobei der Nachweis des ZEBRA-Proteins mit der Sequenz SEQ ID NR.: 1 den folgenden Schritt umfasst:
- Bestimmung des Vorhandenseins eines Immunkomplexes in einer biologischen Probe, die vorher einem für EBV seropositiven Patienten abgenommen wurde, wobei der Immunkomplex aus dem oben genannten Protein besteht, das an die folgenden Liganden gebunden ist: monoklonale Antikörper AZ125, die gegen das Peptid 1-140 des ZEBRA-Proteins mit der Sequenz SEQ ID NR.: 1 gerichtet sind, und monoklonale Antikörper AT130 in Kombination, wobei der Antikörper AZ130 der Antikörper ist, der von dem Hybridom sezerniert wird, das bei der CNCM (Collection Nationale de Culture de Microorganismes, Institut Pasteuer, 25 rue du Docteur Roux, 75724 Paris Cedex 15, Frankreich) gemäß dem Budapester Vertrag am 17. November 2011 unter der Referenz CNCM 1-4554 hinterlegt wurde,
wobei die Menge an ZEBRA-Protein mit der Sequenz SEQ ID NR.: 1, die für eine Lymphoproliferation prognostisch oder diagnostisch ist, insbesondere gleich oder größer als 0,15 Mikrogramm/ml der biologischen Probe ist.

2. Verfahren nach Anspruch 1, wobei die Bindung des ZEBRA-Proteins mit der Sequenz SEQ ID NR.: 1 an einen der Liganden mit Hilfe eines Tests vom Typ Immunoassay, insbesondere vom Typ Sandwich-ELISA, detektiert wird, wobei der Antikörper AT125 auf einer geeigneten Platte adsorbiert wird und der Antikörper AZ130 für die Detektion der Fixierung des ZEBRA-Proteins mit der Sequenz SEQ ID NR.: 1 an dem adsorbierten Antikörper AT125 verwendet wird.

3. Verfahren nach Anspruch 1 oder 2, wobei der Nachweis des ZEBRA-Proteins mit der Sequenz SEQ ID NR.: 1 außerdem die Detektion von anti-ZEBRA-Antikörpern und/oder außerdem die Messung von IL-10 umfasst.

4. Verfahren nach einem der Ansprüche 1 bis 3, wobei der Nachweis des ZEBRA-Proteins mit der Sequenz SEQ ID NR.: 1 außerdem die Messung der EBV-Viruslast, insbesondere durch eine Technik vom Typ PCR, insbesondere unter Verwendung von Primern und Sonden umfasst, deren Nucleinsäuresequenzen in der Gruppe ausgewählt werden, die besteht aus: SEQ ID NR.: 2, SEQ ID NR. : 3, SEQ ID NR. : 4, SEQ ID NR.: 5, SEQ ID NR.: 6, SEQ ID NR.: 7, SEQ ID NR.: 8.

5. Verfahren nach einem der Ansprüche 1 bis 4, wobei der immunologische Status des Patienten zu der Gruppe gehört: immunkompetenter Patient, immunsupprimierter Patient oder immungeschwächter Patient, wobei der immunsupprimierte oder immungeschwächte Patient insbesondere zu der Gruppe gehört, die besteht aus: Patient in Erwartung einer Transplantation, Empfängerpatient einer Transplantation, für einen immunsuppressiven Virus wie HIV seropositiver Patient, oder Patient mit einer genetisch übertragenen Immunschwäche.

6. Verfahren nach Anspruch 5, wobei der Patient immunsupprimiert oder immungeschwächt ist, zur Prognose oder zur Diagnose einer ersten lymphoproliferativen Episode, wobei die Menge an ZEBRA-Protein mit der Sequenz SEQ ID NR.: 1 von 1 bis 100 Mikrogramm/ml der biologischen Probe variiert, wobei die erste lymphoproliferative Episode vor der Initiierung jeder therapeutischen Behandlung eines Lymphoms auftritt, wobei das Verfahren außerdem umfasst:
- die Detektion von anti-ZEBRA-Antikörpern, und
- gegebenenfalls die Messung der EBV-Viruslast, wobei die Viruslast mit einer ersten lymphoproliferativen Episode verbunden ist, die gleich oder größer als 10⁴ Kopien für 150 000 Zellen ist, und bevorzugt in einem Bereich liegt, der von 10⁴ bis 10⁹ Kopien für 150 000 Zellen variiert, und
- gegebenenfalls die Messung von IL-10, wobei die Menge an IL-10, die mit einer ersten lymphoproliferativen Episode verbunden ist, größer als 100 Piktogramm/ml ist, und bevorzugt in einem Bereich liegt, der von 100 bis 250 Piktogramm/ml variiert.

7. Verfahren nach Anspruch 5, wobei der Patient immunsupprimiert oder immungeschwächt ist, zur Prognose oder zur Diagnose einer Reaktivierung der replikativen Form von EBV, welche nach einer Behandlung des Patienten wegen einer ersten Lymphom-Episode auftritt, die mit der Reaktivierung von EBV assoziiert ist, wobei die Menge an ZEBRA-Protein mit der Sequenz SEQ ID NR.: 1, die für die replikative Form von EBV prognostisch oder diagnostisch ist, gleich oder größer als 0,15 Mikrogramm/ml der biologischen Probe ist, und bevorzugt in einem Bereich liegt, der von 0,15 bis 20 Mikrogramm/ml variiert, wobei das Verfahren außerdem umfasst:
- die Detektion von anti-ZEBRA-Antikörpern, und
- gegebenenfalls die Messung der EBV-Viruslast, wobei die EBV-Viruslast, die mit der Reaktivierung der replikativen Form von EBV verbunden ist, gleich oder größer als 10³ Kopien für 150 000 Zellen ist, und bevorzugt in einem Bereich liegt, der von 10³ bis 10⁹ Kopien für 150 000 Zellen variiert.

8. Verfahren nach Anspruch 5, wobei der Patient immunkompetent ist und keine klinischen Anzeichen einer infektiösen Mononucleose zeigt, wobei die Menge an ZEBRA-Protein mit der Sequenz SEQ ID NR.: 1, die für eine Reaktivierung der replikativen Form von EBV prognostisch oder diagnostisch ist, gleich oder größer als 7 Mikrogramm/ml der biologischen Probe ist, wobei das Verfahren außerdem umfasst:
- die Detektion von anti-ZEBRA-Antikörpern, und
- gegebenenfalls die Messung der EBV-Viruslast, wobei die Viruslast, die mit einer Reaktivierung der replikativen Form von EBV verbunden ist, von 10² bis 10³ Kopien für 150 000 Zellen variiert, und
- gegebenenfalls die Messung von IL-10, wobei die Menge an IL-10, die mit einer Reaktivierung der replikativen Form von EBV verbunden ist, größer als 100 pg/ml ist, und bevorzugt in einem Bereich von 100 bis 250 pg/ml liegt.

9. Verfahren nach einem der Ansprüche 1 bis 8, wobei der Nachweis des Vorhandenseins des ZEBRA-Proteins mit der Sequenz SEQ ID NR.: 1 mit der Zeit wiederholt wird, wobei das Zeitintervall, das zwischen jedem Nachweis liegt, höchstens 10 Tage, bevorzugt höchstens 5 Tage und bevorzugter 3 Tage, beträgt.

10. Kit zur Detektion des ZEBRA-Proteins mit der Sequenz SEQ ID NR.: 1, zur Durchführung des Nachweises des Vorhandenseins des ZEBRA-Proteins mit der Sequenz SEQ ID NR.: 1, das gemäß einem der Ansprüche 1 bis 4 definiert ist, wobei das Detektionskit die Antikörper AT125 und AT130 umfasst.

11. Detektionskit nach Anspruch 10, wobei das Detektionskit zusätzlich zu Antikörpern, die spezifisch gegen das ZEBRA-Protein gerichtet sind, umfasst:
- Antigene, welche die Detektion von anti-ZEBRA-Antikörpern, insbesondere des ZEBRA-Proteins in seiner rekombinanten Form oder von Peptiden, die von der Sequenz SEQ ID NR.: 1 abgeleitet sind, gestatten, und/oder
- Mittel zur Detektion der Viruslast, wobei diese Mittel bevorzugt aus Primern bestehen, deren Nucleinsäuresequenz in der Gruppe ausgewählt ist, die besteht aus: SEQ ID NR.: 2 und SEQ ID NR.: 8, und/oder
- Mittel zur Detektion von IL-10, insbesondere anti-IL-10-Antikörpern.

12. Antikörper AZ130, der spezifisch das terminale Fragment C des ZEBRA-Proteins mit der Sequenz SEQ ID NR.: 1 bindet, wobei der Antikörper AZ130 der Antikörper ist, der von dem Hybridom sezerniert wird, das bei der CNCM (Collection Nationale de Culture de Microorganismes, Institut Pasteuer, 25 rue du Docteur Roux, 75724 Paris Cedex 15, Frankreich) gemäß dem Budapester Vertrag am 17. November 2011 unter der Referenz CNCM 1-4554 hinterlegt wurde.

13. Hybridom, welches bei der CNCM (Collection Nationale de Culture de Microorganismes, Institut Pasteuer, 25 rue du Docteur Roux, 75724 Paris Cedex 15, Frankreich) gemäß dem Budapester Vertrag am 17. November 2011 unter der Referenz CNCM 1-4554 hinterlegt wurde, welches den Antikörper AT130, wie in Anspruch 12 definiert, sezernieren kann.

## Claims

1. *In vitro* and/or *ex vivo* prognosis or diagnosis method of lymphoproliferation associated with the EBV replicative form or more generally a tumor associated with EBV, in particular nasopharyngeal cancer, said method requiring the detection of the presence of the ZEBRA protein of sequence SEQ ID NO : 1, in a biological sample selected from the group constituted by blood and serum, from an EBV seropositive patient,
said detection of the ZEBRA protein of sequence SEQ ID NO: 1 comprinsing the following stage :
- Determining the presence of an immune complex in a biological sample collected beforehand from a patient seropositive for EBV, said immune complex being constituted by the aforementioned protein bound to the following ligands : AZ125 monoclonal antibody directed against the peptide 1-140 of the ZEBRA protein of SEQ ID NO: 1 and AZ130 monoclonal antibody used in combination, the AZ130 antibody being the antibody secreted by the hybridoma deposited at the CNCM (Collection Nationale de Culture de Microorganismes, Institut Pasteur, 25 rue du Docteur Roux, 75724 Paris Cedex 15, France) under the Budapest Treaty on 17 November 2011, under the reference CNCM I-4554,
Said quantity of ZEBRA protein of sequence SEQ ID NO: 1 prognosis or diagnosis of a lymphoproliferation being in particular equal to or greater than 0.15 micrograms/ml of biological sample.

2. Method according to claim 1, in which the binding of the ZEBRA protein of sequence SEQ ID NO: 1 to one of the ligands is detected using an immunoassay type test, in particular of the sandwich ELISA type which the AZ125 antibody is absorbed on a suitable plate and the AZ130 antibody is used for detecting the binding of the ZEBRA protein of sequence SEQ ID NO: 1 to the absorbed AZ125 antibody.

3. Method according to claims 1 or 2, in which the detection of the ZEBRA protein of sequence SEQ ID NO: 1 also comprises the detection of anti-ZEBRA antibodies, and/or also IL-10 assay.

4. Method according to one of claims 1 to 3, in which the detection of the ZEBRA protein of sequence SEQ ID NO: 1 also comprises the measurement of the EBV viral load, in particular using primers and probes the nucleic acid sequences of which are selected from the group constituted by SEQ ID NO: 2, SEQ ID NO: 3, SEQ ID NO: 4, SEQ ID NO: 5, SEQ ID NO: 6, SEQ ID NO: 7, SEQ ID NO: 8.

5. Method according to claims 1 to 4, in which the immunological status of the patient belongs to the group : immunocompetent patient, immunosuppressed patient or immunodeficient patient, the aforementioned immunosuppressed patient or aforementioned immunodeficient patient belonging in particular to the group constituted by : patient awaiting a transplant, patient having received a transplant, patient seropositive for an immunosuppressive virus such as HIV, or patient having a genetically transmitted immunological deficiency

6. Method according to claim 5, in which the patient is immunosuppressed or immunodeficient for the prognosis or diagnosis of a first episode of lymphoproliferation, in which the quantity of ZEBRA protein of sequence SEQ ID NO: 1 varies from 1 to 100 micrograms/ml of biological sample, said first episode of lymphoproliferation occurring before the initiation of any therapeutic treatment of a lymphoma, which also comprises :
- the detection of anti-ZEBRA antibodies, and
- optionally the measurement of the EBV viral load, said viral load linked to a first episode of lymphoproliferation being approximately equal to or greater than 10⁴ copies per 150,000 cells, and preferably comprised within a range varying from 10⁴ to approximately 10⁹ copies per 150,000 cells, and
- optionally IL-10 assay, the quantity of IL-10 linked to a first episode of lymphoproliferation being greater than approximately 100 picograms/ml, and preferably comprised within a range varying from 100 to 250 picograms/ml.

7. Method according to claim 5, in which the patient is immunosuppressed or immunodeficient, for the prognosis or diagnosis of a reactivation of the replicative form of EBV occurring following a treatment of the patient for a first episode of lymphoma associated with EBV reactivation, in which the quantity of ZEBRA protein of sequence SEQ ID NO: 1 prognosis or diagnosis of a reactivation of the replicative form of EBV is approximately equal to or greater than 0,15 micrograms/ml of biological sample, and preferably comprised within a range varying from 0,15 to 20 micrograms/ml, which also comprise :
- the detection of the anti-ZEBRA antibodies, and
- optionally the measurement of the EBV viral load, said EBV viral load linked to the reactivation of the replicative form of EBV being approximately equal to or greater than 10³ copies per 150,000 cells, and preferably comprised within a range varying from 10³ to approximately 10⁹ copies per 150,000 cells.

8. Method according to claim 5, in which the patient is immunocompetent and does not present clinical signs of infectious mononucleosis, in which the quantity of ZEBRA protein of sequence SEQ ID NO: 1 prognosis or diagnosis of a reactivation of the replicative form of EBV is approximately equal to or greater than 7 micrograms/ml of biological sample, which also comprises :
- the detection of the anti-ZEBRA antibodies, and
- optionally the measurement of the EBV viral load, said viral load linked to a reactivation of the replicative form of EBV varying from 10² to 10³ copies per 150,000 cells, and
- optionally IL-10 assay, the quantity of IL-10 linked to a reactivation of the replicative form of EBV being greater than approximately 100 pg/ml, and preferably comprised within a range from approximately 100 to approximately 250 pg/ml.

9. Method according to one of claims 1 to 8 in which the detection of the presence of the ZEBRA protein of sequence SEQ ID NO: 1 is repeated over time, the time interval comprised between each detection being 10 days at most, preferably 5 days at most, and more preferably 3 days.

10. Kit for the detection of the ZEBRA protein of sequence SEQ ID NO:1 for implementation of the detection of the presence of ZEBRA protein of SEQ ID NO: 1 according to one of claims 1 to 4, said detection kit comprising antibodies AZ125 and AZ130.

11. Detection kit according to claim 14, said detection kit comprising in addition to the antibodies specifically directed against the ZEBRA protein:
- antigens allowing the detection of the anti-ZEBRA antibodies, in particular the ZEBRA protein in its recombinant form or peptides derived from SEQ ID NO: 1, and/or
- viral load detection means, these means being preferably constituted by primers the nucleic acid sequence of which is selected from the group constituted by: SEQ ID NO: 2 to SEQ ID NO: 8, and/or,
- means for detecting IL-10, in particular anti-IL10 antibodies.

12. AZ130 antibody which specifically binds the C terminal fragment of the ZEBRA protein of sequence SEQ ID NO: 1, the AZ130 antibody being the antibody secreted by hybridoma deposited at the CNCM (Collection Nationale de Culture de Microorganismes, Institut Pasteur, 25 rue du Docteur Roux, 75724 Paris Cedex 15, France) under the Budapest Treaty on 17 November 2011, under the reference CNCM I-4554.

13. Hybridoma deposited at the CNCM (Collection Nationale de Culture de Microorganismes, Institut Pasteur, 25 rue du Docteur Roux, 75724 Paris Cedex 15, France) under the Budapest Treaty on 17 November 2011, under the reference CNCM I-4554, capable of secreting the AZ130 antibody as defined in claim 12.
